(19) Europäisches Patentamt
European Patent Office
Office européen des brevets

(11) EP 0 521 408 B1

(12) **EUROPÄISCHE PATENTSCHRIFT**

(45) Veröffentlichungstag und Bekanntmachung des Hinweises auf die Patenterteilung:
**04.09.1996 Patentblatt 1996/36**

(51) Int. Cl.⁶: **C07K 9/00**, C07K 7/06, C07K 7/52, A61K 38/12, A61K 38/14

(21) Anmeldenummer: **92110823.9**

(22) Anmeldetag: **26.06.1992**

(54) **Balhimycin-abgeleitete glykopeptidische Antibiotika**
Glycopeptide antibiotics derivated from balhimycin
Antibiotiques glycopeptidiques dérivés de la balhimycine

(84) Benannte Vertragsstaaten:
**AT BE CH DE DK ES FR GB GR IT LI LU NL PT SE**

(30) Priorität: **29.06.1991 DE 4121662**
**19.10.1991 DE 4134611**

(43) Veröffentlichungstag der Anmeldung:
**07.01.1993 Patentblatt 1993/01**

(73) Patentinhaber: **HOECHST AKTIENGESELLSCHAFT**
**65926 Frankfurt am Main (DE)**

(72) Erfinder:
- **Vertesy, Laszlo Dr.**
**W-6239 Eppstein/Ts. (DE)**
- **Betz, Joachim Dr.**
**W-6000 Frankfurt am Main (DE)**
- **Fehlhaber, Hans-Wolf Dr.**
**W-6270 Idstein/Ts. (DE)**
- **Limbert, Michael Dr.**
**W-6238 Hofheim am Taunus (DE)**

(56) Entgegenhaltungen:
**EP-A- 0 187 722**

EP 0 521 408 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft neue Glycopeptide, Verfahren zu ihrer Herstellung und ihre Verwendung.

Es ist bereits eine große Anzahl von Glycopeptid-Antibiotika beschrieben worden. Viele dieser Antibiotika sind aber schwächer wirksam als der Urtyp der Glycopeptide und Handelsprodukt Vancomycin und sind diesem insbesondere auch in vivo unterlegen (vgl. R. Nagarajan, Antimicrobial Agents and Chemotherapy, April 1991, Seite 605 - 609).

Das Vancomycin ist zwar einsetzbar bei Infektionskrankheiten, die durch Gram-positive Erreger verursacht werden, eine Reihe von schweren Nebenwirkungen, wie z.B. das sog. "red man syndrom", Nekrosebildungen und andere schränken jedoch die Anwendbarkeit stark ein. Ein weiteres sehr wirksames Glycopeptid-Antibiotikum ist das Balhimycin (vgl. EP 0 468 504, auf die an dieser Stelle ausdrücklich Bezug genommen wird).

Es wurde nun überraschenderweise gefunden, daß mit dem Balhimycin verwandten Verbindungen antibiotisch stark wirksame Substanzen zur Verfügung gestellt werden können, wobei die vom Vancomycin bekannten Nebenwirkungen nicht bzw. in abgeschwächter Form auftreten.

Erfindungsgegenstand sind demzufolge Des-Methyl-Balhimycin eine Verbindung der Formel I,

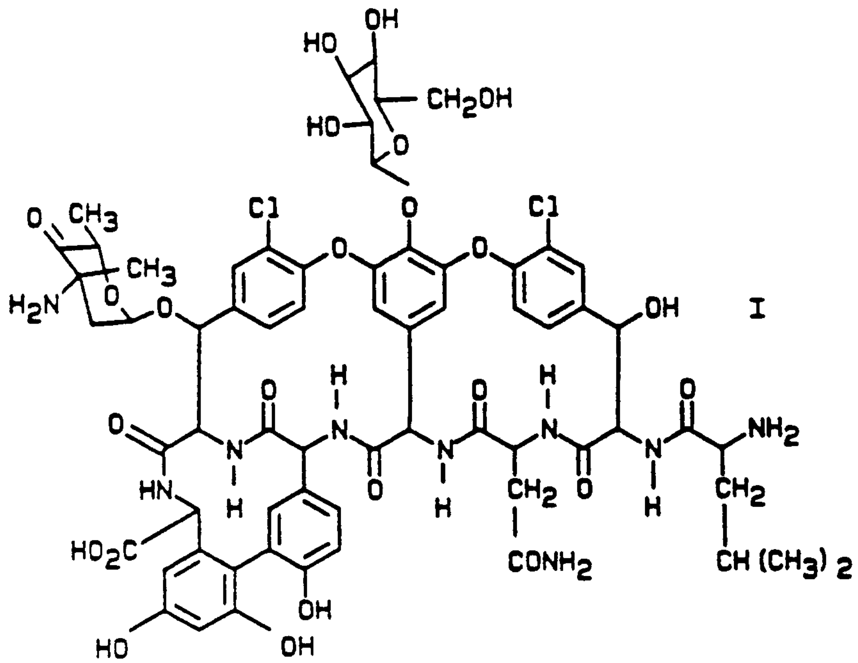

I

Des-Methylleucyl-Balhimycin eine Verbindung der Formel II,

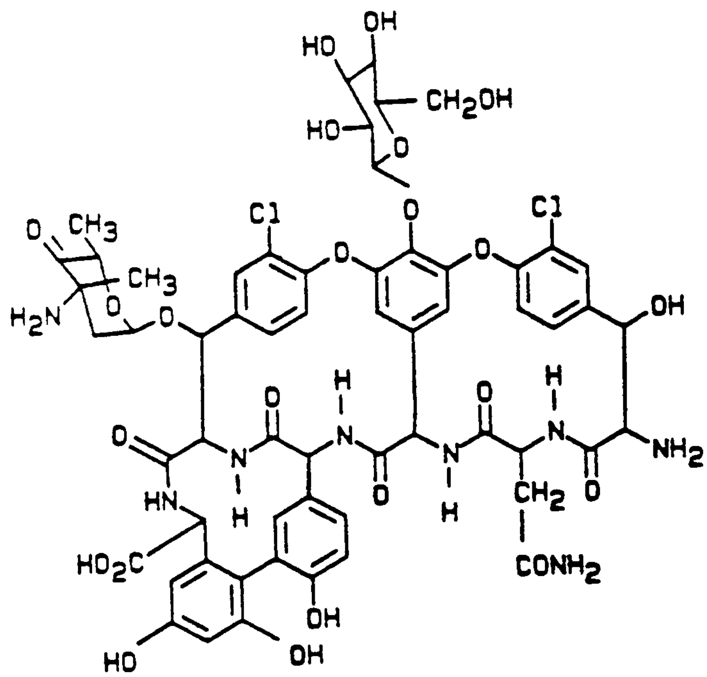

II

Des-Gluco-Balhimycin eine Verbindung der Formel III,

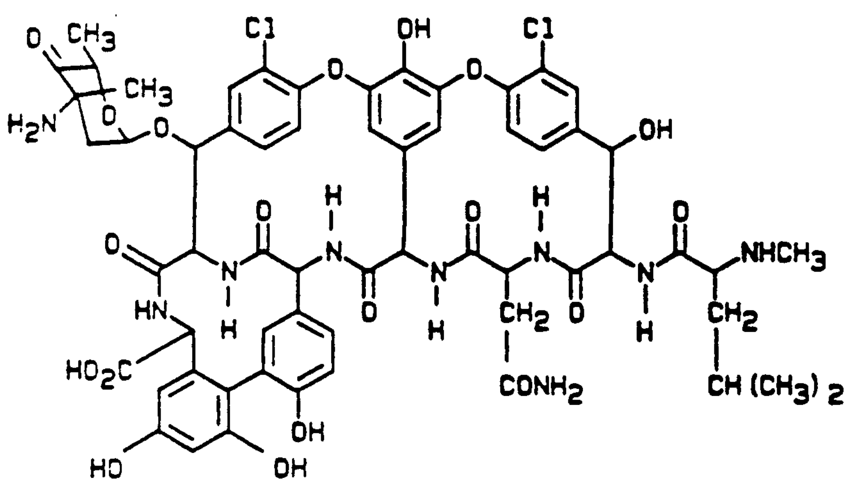

III

Ureido-Balhimycin, eine Verbindung der Formel IV,

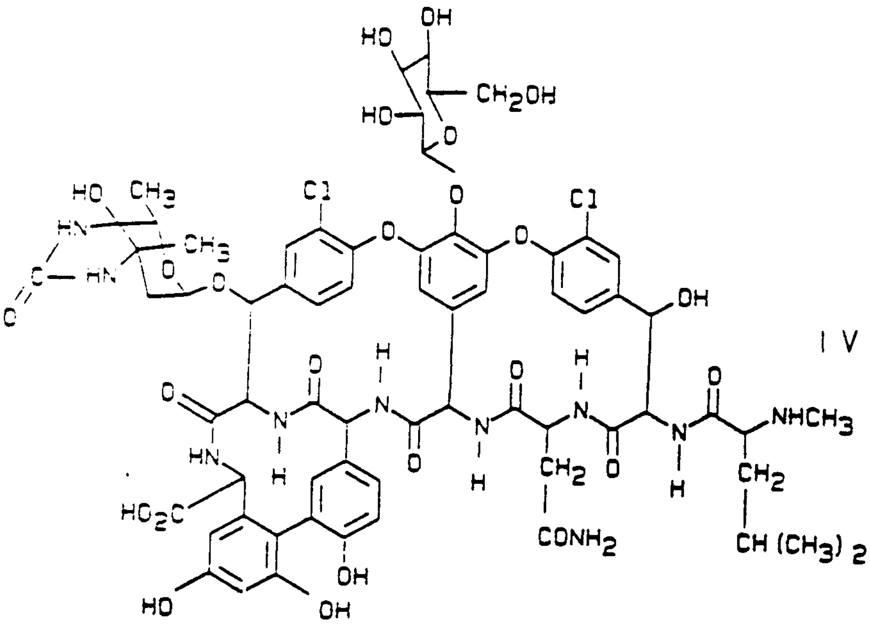

IV

Des-Methyl-des-Gluco-Balhimycin, eine Verbindung der Formel V,

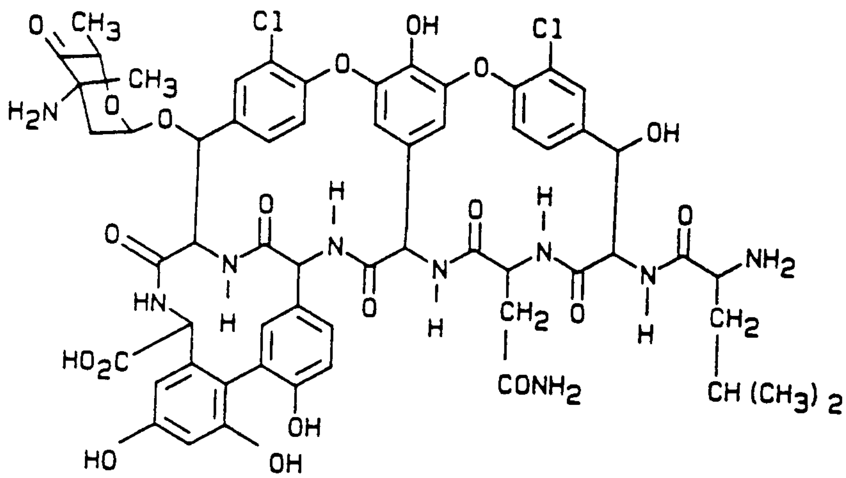

V

Methyl-Balhimycin, eine Verbindung der Formel $C_{67}H_{75}Cl_2N_9O_{24}$,Balhimycin R, eine Verbindung der Formel $C_{72}H_{83}Cl_2N_9O_{28}$ und Balhimycin V, eine Verbindung der Formel $C_{73}H_{84}Cl_2N_{10}O_{26}$ sowie deren Hydrate und physiologisch verträgliche Salze.

Die Hydrate der genannten Verbindungen bilden sich durch Wasseranlagerung wie an dem Beispiel Des-Methyl-Balhimycin nachfolgend dargestellt.

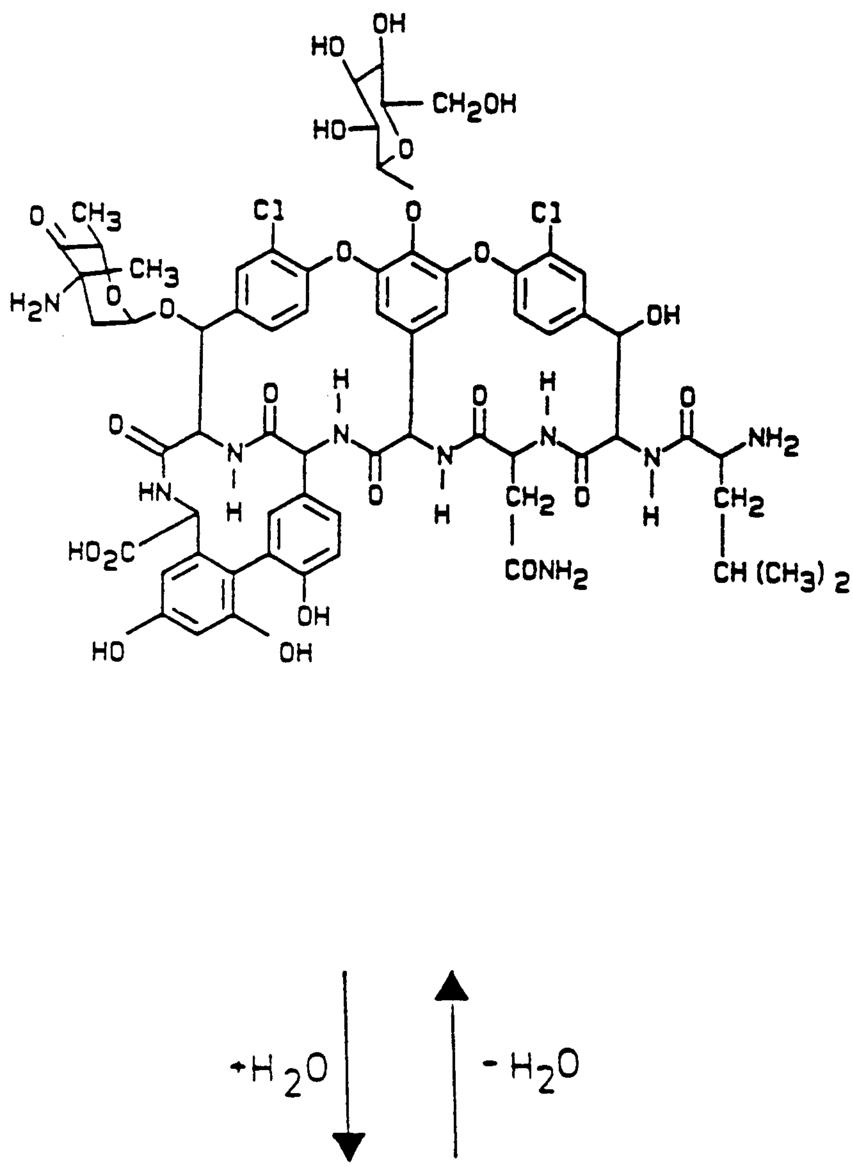

+H2O
- H2O

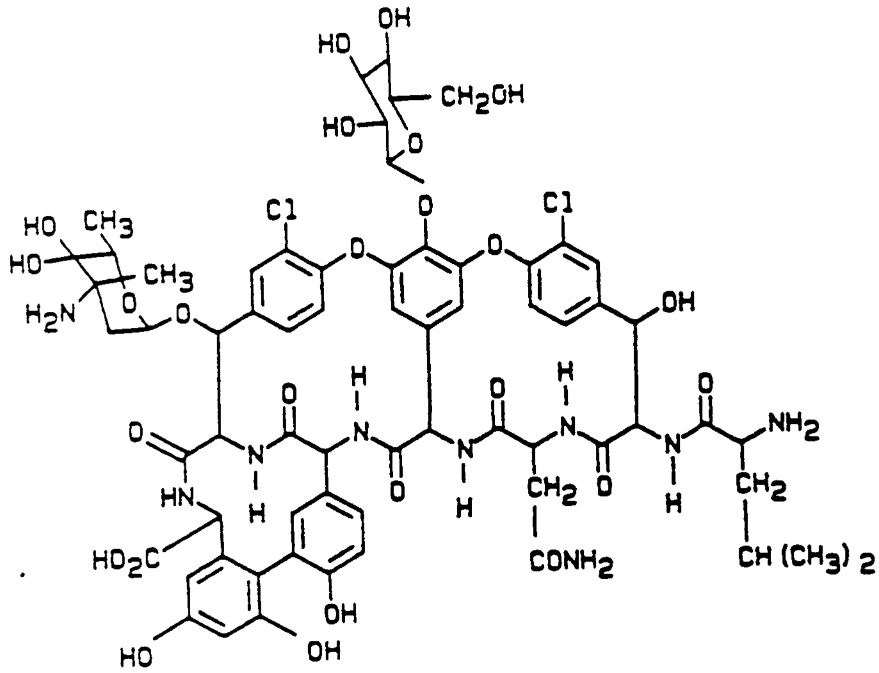

Physiologisch verträgliche Salze der genannten Verbindungen sind z.B. die Acetate, Hydrochloride, Phosphate, Sulphate etc, die sich auf allgemein bekannte Weise erhalten lassen.

Weiterhin gehören zum Gegenstand der vorliegenden Erfindung die Verfahren zur Herstellung der genannten Verbindungen. Ein Verfahren zur Herstellung der genannten Verbindungen ist dadurch gekennzeichnet, daß der Mikroorganismus Actinomyces species Y-86,21022 (DSM 5908) in einem wäßrigen Nährmedium kultiviert wird und die Zielverbindungen anschließend isoliert und gereinigt werden. Der genannte Mikroorganismus ist am 6. April 1990 unter den Bedingungen des Budapester Vertrags hinterlegt worden.

Die Kultivierung des genannten Mikroorganismus erfolgt wie in der obengenannten EP beschrieben in einem wäßrigen Nährmedium enthaltend Kohlenstoffquellen, Stickstoffquellen und mineralische Salze. Bevorzugte Kultivierungsbedingungen sind in der obengenannten EP beschrieben; weitere bevorzugte Bedingungen sind in dem nachfolgenden Beispiel genannt.

Bei der Kultivierung des genannten Mikroorganismus werden hauptsächlich Balhimycin und in nur geringeren Mengen die obengenannten Verbindungen gebildet. Durch Variation der Nährbodenzusammensetzung, insbesondere hinsichtlich der Stickstoffquelle kann erreicht werden, daß von den erfindungsgemäßen Verbindungen deutlich größere Mengen gebildet werden. So ist überraschenderweise gefunden worden daß die Gabe von millimolaren Konzentrationen an Methionin, Serin und Pyruvat die Bildung von Balhimycin unterdrückt. Setzt man Antagonisten des Methionin, wie z. B. 1 mM $\alpha$-Methyl-Methionin ein, kommt es zu einer deutlichen Steigerung der Ausbeuten mit stärkerer Betonung der Des-Methyl-Komponente des Balhimycins. Allosterische Inhibitoren des Aspartatmetabolismus, wie L-Lysin oder L-Threonin sowie Antagonisten des Leucin wirken sich ebenfalls auf das Produktspektrum aus.

Darüber hinaus kann das Produktspektrum des Stammes Actinomyces species Y-86,21022 durch genetische Maßnahmen beeinflußt werden. Mutationen mit an sich bekannten Mutagenen physikalischer oder chemischer Art in Kombination mit geeigneten Selektionsmethoden, z. B. Antimetabolitresistenz, führen zu Mutanten, die die gewünschten Nebenkomponenten in stark erhöhtem Anteil oder ausschließlich produzieren.

Die Trennung der genannten Glycopeptide erfolgt vorzugsweise mittels Kationenaustauschern in Puffersystemen mit einem hohen Anteil an organischen Lösungsmitteln. Geeignete Lösungsmittel sind z.B. mit Wasser mischbare organische Lösungsmittel wie niedere Alkohole, Aceton, Acetonitril, Glykol, Dioxan, Dimethylsulfoxid, Formamid und ähnliche aber auch wässrige Harnstofflösungen. Bevorzugte Lösungsmittel sind Methanol, Ethanol, Isopropanol und Aceton. Besonders geeignete Lösungsmittelanteile sind 5 - 95 % organische Lösungsmittel in den wässrigen Pufferlösungen, besonders bevorzugte Anteile liegen zwischen 25 und 85 %. Da der Trenneffekt sich mit steigendem Lösungsmittelanteil eher verbessert, arbeitet man in der Praxis zweckmäßigerweise mit einem Anteil an organischem Lösungsmittel nicht unter 35 %.

Eine andere Möglichkeit der Trennung in technisch durchführbarem Maßstab besteht darin, daß man "Reverse Phasen" verwendet und durch geeignete Maßnahmen die Trennschärfe verbessert. Solche Maßnahmen sind die Verwendung von Zusätzen wie Salzen, z. B. Phosphatpuffer und anderen oder von chaotrophen Substanzen wie Harnstoff, $KClO_4$ oder weitere Agenzien wie Komplexbildner, Ionenpaarbildner u. a. in den Elutionsmitteln.

Ein alternativer Schritt zur Isolierung der erfindungsgemäßen Verbindungen ist die Kristallisation. Hierbei wird die Kristallisationsneigung der erfindungsgemäßen Verbindungen in der Nähe des isoelektrischen Punktes, ihre Abhängigkeit von Lösungsmittel-Beimengungen in der Mutterlauge und von der Art der Gegenionen ausgenutzt. Z. B. lassen sich in wäßriger Lösung befindliche erfindungsgemäße Verbindungen durch Zugabe wasserlöslicher, organischer Lösungsmittel wie z. B. Ethanol oder Isopropanol zur Kristallisation bringen.

Oder die in wäßrig saurer Lösung vorliegenden Verbindungen werden durch Erhöhung des pH-Wertes, z. B. mittels Zugabe von $NH_3$ zur Kristallisation gebracht. Die erhaltenen kristallinen Verbindungen, wie z. B. das Ureido-Balhimycin, das in der nichtcentrosymmetrischen Raumgruppe P1 mit 2 Molekülen in der Elementarzelle und den Zellkonstanten a = 17,909 Å, b = 18,466 Å, c = 18,873 Å, $\alpha$ = 96,65, $\beta$ = 114,15, $\gamma$ = 114,78° kristallisiert, gehören ebenfalls zu der vorliegenden Erfindung.

Ein weiteres Verfahren zur Herstellung von Des-Methylleucyl-Balhimycin besteht darin, daß mit Balhimycin oder mit Des-Methyl-Balhimycin ein Edman-Abbau durchgeführt wird (vgl. "Practical Protein Chemistry, A Handbook" A. Darbre, Seite 345 ff., John Wiley & Sons, 1987).

Ein zusätzliches Verfahren zur Herstellung des Des-Gluco-Balhimycins ist dadurch gekennzeichnet, daß mit Balhimycin eine hydrolytische Spaltung durchgeführt wird. Ein besonders bevorzugtes Hydrolyse-Agens ist 4n Trifluoressigsäure oder höherkonzentrierte, insbesondere auch bei leicht erhöhter Temperatur.

Ein zusätzliches Verfahren zur Herstellung von Des-Methyl-des-Gluco-Balhimycin ist dadurch gekennzeichnet, daß mit Des-Methyl-Balhimycin eine hydrolytische Spaltung durchgeführt wird. Ein besonders bevorzugtes Hydrolyse-Agens ist 4n oder höher konzentrierte Trifluoressigsäure, insbesondere bei Raumtemperatur oder leicht erhöhter Temperatur.

Ein zusätzliches Verfahren zur Herstellung von Ureido-Balhimycin ist dadurch gekennzeichnet, daß Balhimycin mit Isocyanaten wie z. B. Kalium-Isocyanat oder mit Harnstoff umgesetzt wird. Man kann diese Reaktion zum Beispiel in wäßriger Lösung in einem weiten pH-Bereich durchführen, vorzugsweise in dem Bereich zwischen pH 4 und 8.

Die erfindungsgemäßen neuen Verbindungen sind mit dem Glykopeptid-Antibiotikum Balhimycin nahe verwandt und leiten sich strukturell von diesem ab. Sie lassen sich im einzelnen wie folgt charakterisieren:

a) Des-Methyl-Balhimycin ist dadurch gekennzeichnet, daß die Verbindung durch den Stamm Y-86,21022 (DSM 5908) gebildet wird und folgende Eigenschaften hat:

| | |
|---|---|
| Summenformel: | $C_{65}H_{71}Cl_2N_9O_{24}$<br>bestimmt durch FAB-Massenspektrometrie:<br>$M + H^+ = 1432,4$<br>für das Isotop: $^{12}C_{65}{}^{1}H_{71}{}^{35}Cl_2{}^{14}N_9{}^{16}O_{24}$ |
| Chemisches Molekulargewicht: | 1433,25 Da<br>Aminosäuren-Analyse (nach Hydrolyse in 5 M Salzsäure bei 100°C, 20 Stunden):<br>Asparaginsäure,<br>Leucin, neben anderen ungewöhnlichen<br>Ninhydrin-positiven Substanzen<br>UV-Maxima: 281 nm (log E 3,8) |

Das Desmethyl-Balhimycin unterscheidet sich also vom Balhimycin dadurch, daß es Leucin an Stelle von N-Methyl-Leucin enthält.

b) Des-Methylleucyl-Balhimycin ist dadurch gekennzeichnet, daß es mittels des Stammes Y-86,21022 (DSM 5908) produziert wird und daß es folgende Eigenschaften hat:

| | |
|---|---|
| Summenformel | $C_{59}H_{60}Cl_2N_8O_{23}$<br>bestimmt durch FAB-Massenspektrometrie:<br>$M + H^+ = 1319,3$<br>für das Isotop: $^{12}C_{59}{}^{1}H_{60}{}^{35}Cl_2{}^{14}N_8{}^{16}O_{24}$ |
| Chemisches Molekulargewicht: | 1320,08 Da<br>Aminosäuren-Analyse (nach Hydrolyse in 5 M Salzsäure bei 100°C, 20 Stunden):<br>Asparaginsäure,<br>neben ungewöhnlichen Ninhydrin-positiven Substanzen.<br>Es fehlen: Leucin und N-Methylleucin |
| UV-Maxima: | 281 nm, (log E 3,8) |

Das Des-Methylleucyl-Balhimycin unterscheidet sich vom Balhimycin dadurch, daß das N-Methylleucin fehlt.

c) Des-Gluco-Balhimycin ist dadurch gekennzeichnet, daß es vom Actinomyceten-Stamm Y-86,21022 (DSM 5908) gebildet wird, und folgende Eigenschaften hat:

| | |
|---|---|
| Summenformel: | $C_{60}H_{63}Cl_2N_9O_{19}$ |
| | bestimmt durch FAB-Massenspektrometrie: |
| | $M + H^+ = 1284,4$ |
| | für das Isotop: $^{12}C_{60}\,^{1}H_{63}\,^{35}Cl_2\,^{14}N_8\,^{16}O_{19}$ |
| Chemisches Molekulargewicht: | 1285,12 Da |
| | Aminosäuren-Analyse (nach Hydrolyse in 5 M Salzsäure bei 100°C, 20 Stunden): |
| | Asparaginsäure, |
| | N-Methylleucin |
| | neben ungewöhnlichen Ninhydrin-positiven Substanzen |
| UV-Maxima: | 279 nm, (log $\varepsilon$ : 3,8) |

Das Des-Gluco-Balhimycin unterscheidet sich gegenüber dem Balhimycin durch das Fehlen eines Glucose-Restes.

d) Ureido-Balhimycin ist dadurch gekennzeichnet, daß es vom Actinomycetenstamm Y-86,21022 (DSM 5908) gebildet wird und folgende Eigenschaften hat:

| | |
|---|---|
| Summenformel: | $C_{67}H_{74}Cl_2N_{10}O_{25}$ |
| | bestimmt durch FAB-Massenspektrometrie: |
| | $M + H^+ = 1489,4$ |
| | für das Isotop: $^{12}C_{67}\,^{1}H_{74}\,^{35}Cl_2\,^{14}N_{10}\,^{16}O_{25}$ |
| Chemisches Molekulargewicht: | 1490,29 Da. |
| UV-Maxima | 280 nm (log. $\varepsilon$ : 3,8) |

Das Ureido Balhimycin ist das an den C-Atomen 3 und 4 des Dehydrovancosamins liegende cyclische Ureid des Antibiotikums Balhimycin.

e) Methyl-Balhimycin ist dadurch gekennzeichnet, daß es vom Actinomycetenstamm Y-86,21022 (DSM 5908) gebildet wird und folgende Eigenschaften hat:

| | |
|---|---|
| Summenformel: | $C_{67}H_{75}Cl_2N_9O_{24}$ |
| | bestimmt durch FAB-Massenspektrometrie: |
| | $M + H^+ = 1460,45$ |
| | für das Isotop: $^{12}C_{67}\,^{1}H_{75}\,^{35}Cl_2\,^{14}N_9\,^{16}O_{24}$ |

f) Balhimycin R ist dadurch gekennzeichnet, daß es vom Actinomycetenstamm Y-86,21022 (DSM 5908) gebildet wird und folgende Eigenschaften hat:

| | |
|---|---|
| Summenformel: | $C_{72}H_{83}Cl_2N_9O_{28}$ |
| | bestimmt durch FAB-Massenspektrometrie: |
| | $M + H^+ = 1592,48$ |
| | für das Isotop: $^{12}C_{72}\,^{1}H_{83}\,^{35}Cl_2\,^{14}N_9\,^{16}O_{24}$ |
| Chemisches Molekulargewicht: | 1593,41 Da. |
| UV-Maxima | 280 nm (log. $\varepsilon$ = 3,8) |

Das Balhimycin R unterscheidet sich gegenüber dem Balhimycin durch einen zusätzlichen Rhamnosyl-Rest.

g) Des-Methyl-des-Gluco-Balhimycin ist dadurch gekennzeichnet, daß es vom Actinomycetenstamm Y-86, 21022 (DSM 5908) gebildet wird und folgende Eigenschaften hat:

| | |
|---|---|
| Summenformel: | $C_{59}H_{61}Cl_2N_9O_{19}$, |
| | bestimmt durch FAB-Massensspektrometrie: |
| | $M + H^+ = 1270,35$ |
| | für das Isotop: $^{12}C_{59}\,^{1}H_{61}\,^{35}Cl_2\,^{14}N_9\,^{16}O_{19}$. |
| Chemisches Molekulargewicht: | 1271,09 |
| UV-Maxima: | 280 nm (log $\varepsilon$ = 3.8). |

h) Balhimycin V ist dadurch gekennzeichnet, daß es vom Actinomycetenstamm Y-86, 21022 (DSM 5908) gebildet wird und folgende Eigenschaften hat:

| | |
|---|---|
| Summenformel: | $C_{73}H_{84}Cl_2N_{10}O_{26}$<br>bestimmt durch FAB-Massenspektrometrie:<br>$M + H^+ = 1587,50$<br>für das Isotop: $^{12}C_{73}{}^1H_{84}{}^{35}Cl_2N_{10}O_{26}$ |
| Chemisches Molekulargewicht: | 1588,44 Da. |
| UV-Maximum: | 280 nm (log $\varepsilon$ = 3.8). |

Das Balhimycin V unterscheidet sich gegenüber dem Balhimycin durch einen zusätzlichen 4-Dehydro-Vancosaminyl-Rest.

Die erfindungsgemäßen Verbindungen sind farblose, in Wasser oder in wässrigen Lösungen lösliche Substanzen, die in Form eines Feststoffes oder in Lösung vergleichsweise überraschend stabil sind.

Die folgende Tabelle 1 zeigt einige biologische Daten: Minimale bakteriostatische Hemmkonzentrationen in Mikrogramm pro Milliliter bestimmt mittels der Agar-Verdünnungsmethode:

| Tabelle 1 | | Desmethyl-Balhimycin | Des-Methylleucyl-Balhimycin | Desgluco Balhimycin |
|---|---|---|---|---|
| Staph. aureus | SG 511 | 0,1 | 3 | 0,2 |
| Staph. aureus | 285 | 0,1 | 6 | 0,2 |
| Staph. aureus | 503 | 0,05 | 6 | 0,1 |
| Strept. pyogenes | 308 A | 0,05 | 12,5 | 0,1 |
| Strept. pyogenes | 77 A | 0,05 | 3 | 0,1 |
| Strept. faecium | D | 0,2 | 6 | 0,2 |
| Escherichia coli | DC 2 | 10 | >100 | >100 |

| | | | | |
|---|---|---|---|---|
| Bact. fragilis | 312 | 100 | 50 | 25 |
| Bact. fragilis | 960 | 25 | 50 | 25 |
| Bact. fragilis | 1313 | 50 | 100 | 25 |
| Bact. vulgatus | 1446 | 50 | 100 | >100 |
| Peptostrept. anaerob. | 932 | 25 | 50 | 0,4 |
| Propioni acnes | 6916 | 0,8 | 6 | 0,2 |
| Propioni acnes | 6922 | 0,4 | 6 | 0,2 |
| Clostridium tetani ATCC 1940650 | | 50 | 50 | 0,4 |
| Clostridium perfringens | 194 | 0,2 | 6 | 0,1 |

| | | Ureido-Balhimycin | Methyl-Balhimycin | Balhimycin R |
|---|---|---|---|---|
| Staph. aureus | SG 511 | 0,8 | 0,4 | 0,2 |
| Staph. aureus | 285 | 1,5 | 0,4 | 0,8 |
| Staph. aureus | 503 | 1,5 | 0,4 | 0,2 |
| Strept. pyogenes | 308A | 0,8 | 0,4 | 0,2 |
| Strept. pyogenes | 77 A | 0,8 | 0,4 | 0,2 |
| Strept. faecium | D | 1,5 | 0,8 | 0,4 |
| Escherichia coli | DC 2 | >100 | 25 | 50 |
| Bact. fragilis | 312 | 25 | 100 | >100 |
| Bact. fragilis | 960 | 25 | 100 | >100 |
| Bact. fragilis | 1313 | 25 | 50 | >100 |
| Bact. vulgatus | 1446 | 50 | >100 | >100 |
| Peptostrept. anaerob. | 932 | 6,2 | 0,8 | 100 |
| Propioni acnes | 6916 | 0,8 | 0,4 | 6,2 |
| Propioni acnes | 6922 | 1,5 | 0,4 | 3,1 |
| Clostridium tetani ATCC 1940650 | | 12,5 | 3,1 | 100 |
| Clostridium perfringens | 194 | 0,4 | 1,5 | - |

| | | Desmethyl-Desgluco-Balhimycin | Balhimycin V |
|---|---|---|---|
| Staph. aureus | SG 511 | 0,2 | 0,4 |
| Staph. aureus | 285 | 0,2 | 0,4 |
| Staph. aureus | 503 | 0,1 | 0,1 |
| Strept. pyogenes | 308 A | 0,1 | 0,05 |
| Strept. pyogenes | 77 A | 0,1 | 0,05 |
| Strept. faecium | D | 0,2 | 0,2 |
| Escherichia coli | DC 2 | >100 | >100 |
| Bact. fragilis | 312 | 50 | n. t. |
| Bact. fragilis | 960 | 25 | n. t. |
| Bact. fragilis | 1313 | 50 | n. t. |
| Bact. vulgatus | 1446 | >100 | n. t. |
| Peptostrep. anaerob. | 932 | 0,2 | n. t. |
| Propioni acnes | 6916 | 0,2 | n. t. |
| Propioni acnes | 6922 | 0,1 | n. t. |
| Clostridium tetani ATCC 19406 | | n. t. | n. t. |
| Clostridium perfringens | 194 | 0,1 | n. t. |

Wie aus der Tabelle 1 ersichtlich, haben die erfindungsgemäßen Verbindungen insbesondere eine hervorragende Wirkung gegen Gram-positive Bakterien einschließlich der sog. Methicillin-resistenten Staphylococcus aureus-Stämme (MRSA). Sie eignen sich deshalb insbesondere zur Behandlung von Infektions-Krankheiten, die durch solche Keime verursacht worden sind. Zum Erfindungsgegenstand gehören demzufolge auch Arzneimittel enthaftend eine wirksame Menge einer erfindungsgemäßen Verbindung sowie die Verwendung der Verbindungen zur Herstellung von Arzneimitteln insbesondere von Arzneimitteln mit antibiotischer Wirkung; die genannte Herstellung erfolgt in herkömmlicher, allgemein bekannter Weise.

Weiterhin eignen sich die erfindungsgemäßen Verbindungen auch zum Einsatz als Wachstumsförderer in der Landwirtschaft.

Durch die nachfolgenden Beispiele und den Inhalt der Patentansprüche soll die vorliegende Erfindung näher erläutert werden.

Beispiel 1: Fermentation der Balhimycin-Komponenten

Als Hauptkultur der Fermentation dient die Nährlösung (NL 5276). Sie setzt sich wie folgt zusammen.

| | | |
|---|---|---|
| NL 5276: | Glycerin 99% | 20 g/l aqua dest |
| | Sojapepton HySoy T | 10 g/l |
| | Glucose | 5 g/l |
| | $CaCO_3$ | 3 g/l |
| | Hefeextrakt, Oxoid | 3 g/l |
| | pH vor Sterilisation | 7,0 |

Die Substrate, außer Glucose, werden unter Rühren in 10 l Wasser eingetragen und auf ein Volumen von 18 l aufgefüllt. Der pH-Wert vor der Sterilisation wird mit verdünnter, 20 - 30 %iger, NaOH auf 7,0 eingestellt. Die im Ansatz vorgeschriebene Menge Glucose wird separat in 1 l Wasser gelöst und 20 Minuten bei 120°C im Autoklaven sterilisiert und dem sterilisierten Ansatz nach Abkühlung zugesetzt. Sterilisiert wird 45 Minuten bei 120 °C und 1,2 - 1,4 bar. Nach dem Abkühlen auf Betriebstemperatur und der Zugabe der Glucoselösung beträgt das Fermentationsvolumen ca. 20 l bei einem pH-Wert von ca 7,0.
Der C-Fermenter wird mit 500 - 1000 ml Vorkultur beimpft, die wie in der EP 0 468 504 Beispiele 2 und 3 hergestellt wird.

| | | |
|---|---|---|
| Fermentationsbedingungen: | Fermentationstemperatur | 28°C |
| | Belüftung | 20 l/Minute = 1 vvm |
| | Druck | 0,5 bar |
| | Drehzahl | 250 Upm |

Als Antischaummittel werden bei Bedarf 5 ml, entsprechend 0,025 % bezogen auf das Fermentationsvolumen, an $^R$Desmophen 3600 (Polyole, Bayer AG, Leverkusen) als steriles Wasser-Desmophengemisch zugegeben.
Die Fermentationszeit beträgt 96 - 120 Stunden. Der pH-Wert wird während der Fermentation nicht korrigiert, jedoch die Kultur auf Sterilität, Stickstoffverbrauch und die Produktbildung durch HPLC untersucht. Danach wird abgeerntet und die Zellmasse durch Zentrifugieren entfernt.

Beispiel 2: Isolierung des Balhimycin-Komplexes

10 l Filtrat der nach Beispiel 1 gewonnenen Kulturen werden auf eine vorbereitete 1 l $^R$Diaion HP-20 (Mitsubishi Chem. Ind.) fassende Säule aufgetragen. Danach wäscht man den beladenen Träger mit entsalztem Wasser. Die Balhimycin-Komponenten werden nun mit einem 0 - 50 % Isopropanol enthaltenden Gradienten eluiert und der Säulenausfluß fraktioniert aufgefangen. Die Fraktionen untersucht man auf antibiotische Wirksamkeit und bestimmt die Komponentenzusammensetzung mittels HPLC. Zunächst werden Des-Methyl-Balhimycin-haltige (I), dann Balhimycin-(II) und schließlich Des-Methylleucyl-Balhimycin-reichere (III) Fraktionen erhalten. Sie werden getrennt gesammelt und ergeben nach Einengen und Gefriertrocknung 650 mg I, 1,1 g II und 380 mg III.

Beispiel 3: Ionenchromatographische Isolierung des Des-Methyl-Balhimycins

Eine 100 ml Chromatographie-Säule wird mit $^R$Fractogel EMD-$SO_3$-Kationenaustauscher gefüllt und mit 25 mM Natrium-Acetatpuffer in 66 % Methanol auf pH 4,8 (Puffer A) eingestellt. Dann werden 650 mg Des-Methyl-Balhimycin-haltiges Antibiotikum, erhalten z. B. entsprechend Beispiel 2 in ungefähr 100 ml Puffer A gelöst, auf die Säule aufgetragen und mit 100 ml Puffer A gewaschen. Im Durchlauf und im Waschwasser befindet sich das antibiotisch aktive, literaturbekannte Des-Dehydro-Vancosamin-Derivat.
Anschließend legt man einen 0 - 200 mM Natriumchloridgradienten in Puffer A, pH 5,0 an. Mit 130 - 150 mM NaCl wird das Balhimycin vom Ionenaustauscher eluiert, mit der 160 - 175 mM NaCl-Lösung das Des-Methyl-Balhimycin.

Die entsprechenden Fraktionen werden jeweils gegen 1/100 M Essigsäure dialysiert und gefriergetrocknet. Das Kristallisieren aus wässriger Lösung unter Zugabe von Ethanol führt zu 210 mg Balhimycinacetat in 98 %iger Reinheit und 160 mg Des-Methyl-Balhimycinacetat in 97 %iger Reinheit.

Hochdruckflüssigkeitschromatograpie (HPLC) Daten:

| | |
|---|---|
| Träger: | $^R$Lichrospher RP18, 5 µm, 250 x 4 $mm^2$ |
| Elutionssystem: | 14 % Acetonitril in 0,1 %iger wässriger Trifluoressigsäure |
| Detektion: | UV-Absorption bei 210 nm |
| Retentionszeit: | 8,3 Minuten, Vergleich Balhimycin: 10,0 Minuten |
| $[\alpha]_D^{22}$: | - 77 ± 2° |

Beispiel 4: Reindarstellung von Des-Methylieucyl-Balhimycin

300 mg des nach Beispiel 2 gewonnenen Des-Methylleucyl-Balhimycin-haltigen Produkts III werden entsprechend dem Beispiel 2 nochmal auf 100 ml MCI-Gel CHP20P (Mitsubishi Chem. Ind.) nachgereinigt, als Puffer A dient jedoch 10 mM $K_2HPO_4$-Puffer, pH 7,6, als Puffer B verwendet man 10 mM $K_2HPO_4$, pH 7,6 in 40 %igem Methanol. Die im Gradientenverfahren durchgeführte Elution ergibt Fraktionen, die mittels HPLC analysiert werden. Die Des-Methylleucyl-Balhimycin-haltigen Fraktionen, mit einer Reinheit von über 90 %, werden vereinigt, im Vakuum eingeengt und auf reverse Phase RP18, im 0,05 % Trifluoressigsäure/Acetonitrilsystem, entsalzt. Die Gefriertrockung der Hauptfraktionen ergibt 120 mg Des-Methylleucyl-Balhimycin-Trifluoracetat in 98 %iger Reinheit.

Hochdruckflüssigkeitschromatographie (HPLC) Daten:

| | |
|---|---|
| Träger: | $^R$Lichrospher RP18, 5 µm, 250 x 4 $mm^2$ |
| Elutionsmittel: | 14 % Acetonitril in 0,1 %iger wässriger Trifluoressigsäure |
| Detektion: | UV-Absorption bei 210 nm |
| Retentionszeit: | 16,0 Minuten, Vergleich Balhimycin: 10,0 Minuten |
| $[\alpha]_D^{22}$: | + 27 ± 2° |

Beispiel 5: Gewinnung des Des-Gluco-Balhimycins und des Des-Methyl-des-Gluco-Balhimycins

300 mg des nach Beispiel 2 gewonnenen Produktes III werden in Wasser gelöst und auf eine präparative, 500 ml fassende HPLC-Säule (250 - 2"), aufgetragen, die mit dem Träger $^R$Nucleosil 1015 C18 P (Macherey-Nagel, Düren) gefüllt ist. Dann wird im Gradientenverfahren in 0,1 % Trifluoressigsäure mit 0 - 20 % Acetonitril eluiert. Während zunächst mit 8 - 10 % Lösungsmittelanteil die Antibiotika Balhimycin und Des-Methylleucyl-Balhimycin vom Träger gelöst werden, wird mit 14 - 15 % Acetonitrilanteil das Des-Methyl-des-Gluco-Balhimycin und das Des-Gluco-Balhimycin gewonnen. Die Gefriertrocknung der Des-Methyl-des-Gluco- bzw. der Des-Gluco-Balhimycin-haltigen Fraktionen und ihre Rechromatographie im gleichen System ergeben 1,3 mg Des-Methyl-des-Gluco-Balhimycin-Trifluoracetatsalz 4 mg Des-Gluco-Balhimycin-Trifluoracetatsalz.

Beispiel 6: Hydrolytischer Abbau des Balhimycins zu Des-Gluco-Balhimycin

5 g Balhimycin, gewonnen entsprechend der Anmeldung EP 0 468 504, Beispiel 4, werden in 120 ml 4 molarer Trifluoressigsäure gelöst und über Nacht bei 45°C reagieren gelassen. Nach dieser Zeit entfernt man das Lösungsmittel unter Vakuum und dann durch Gefriertrockung. Der so konzentrierte Reaktionsansatz wird nun in Wasser gelöst und auf 800 ml MCI-Gel CHP20P (Mitsubishi Chem. Ind.) mit dem Gradienten System 0,1 % Essigsäure/0,1 % Essigsäure in 50 %igem Isopropanol aufgetrennt. Es werden zunächst Balhimycin und Des-Amido-Balhimycin von der Säule eluiert, anschließend Des-Gluco-Balhimycin und zum Schluß Des-Amido-des-Gluco-Balhimycin. Die gewünschten Fraktionen mit einem Reinheitsgrad von über 90 % werden gesammelt, rechromatographiert und gefriergetrocknet. Sie ergeben 1,3 g Des-Gluco-Balhimycinacetat in einer Reinheit von 98,5 %.

Hochdruckflüssigkeitschromatographie (HPLC) Daten:

| | |
|---|---|
| Träger: | ®Lichrospher RP18, 5 µm, 250 x 4 $mm^2$ |
| Elutionsmittel: | 19 % Acetonitril in 0,1 %iger wässriger Trifluoressigsäure |
| Detektion: | UV-Absorption bei 210 nm |
| Retentionszeit: | 10,0 Minuten |
| $[\alpha]_D^{22}$ : | - 70,5 ± 2° |

Beispiel 7: Gewinnung des Ureido-Balhimycins

1 g Roh-Balhimycin (II), gewonnen entsprechend Beispiel 2, werden in 25 ml Wasser gelöst, der pH-Wert mit Essigsäure auf 3,5 gestellt und auf eine vorbereitete, bei pH 3,5 äquilibrierte, 150 ml Fractogel EMD-$SO_3$ Kationenaustauscher fassende Säule aufgetragen.

Nach dem Auftragen wird zunächst mit 200 ml reinem Wasser, dann mit 200 ml 25 mM Natriumacetat-Puffer pH 4,0 (Puffer A) nachgewaschen.

Der Säulenausfluß dieser Pufferlösung enthält das Ureido-Balhimycin, Eluat WP genannt. Anschließend wird die Säule durch Anlegen eines 0,1 M NaCl Gradienten in 25 mM Natriumacetat, pH 4,0 eluiert. Mit 20 - 30 mM NaCl wird Balhimycin R (Eluat R) mit 30 - 70 mM überwiegend Balhimycin (Eluat B) und 80 - 100 mM NaCl Des-Methyl- sowie Methyl-Balhimycin (Eluat M) gewonnen.

Zur Reindarstellung des Ureido-Balhimycins wird das Eluat WP auf eine Nucleosil 10-$C_{18}$ AB reverse Phase Säule (20 mm ID x 250 mm Höhe) gegeben und im Gradienten-Verfahren mit dem 0,1 % Trifluoressigsäure/Acetonitrilsystem - wie im Beispiel 5 geschildert - aufgetrennt. Die Gefriertrocknung der Ureido-Balhimycinhaltigen Fraktionen ergibt 20 mg dieses Antibiotikums als Trifluoracetat-Salz.

Hochdruckflüssigkeitschromatographie (HPLC) Daten:

| | |
|---|---|
| Träger: | ®Lichrospher RP18, 5 µm, 250 x 4 $mm^2$ |
| Elutionsmittel: | 14 % Acetonitril in 0,1 %iger wässriger Trifluoressigsäure |
| Detektion: | UV-Absorption bei 210 nm |
| Retentionszeit: | 13,5 min, Vergleich Balhimycin: 10,0 min |
| $[\alpha]_D^{24}$ = | - 26 ° (c = 1 % in Wasser) |

Beispiel 8: Gewinnung des Methyl-Balhimycins

Das Eluat M, gewonnen nach Beispiel 7, wird auf der 20 mm x 250 mm (ID x H) ®Nucleosil 10-$C_{18}$ AB-Säule gereinigt mit Hilfe des Gradientensystems 10 mM $K_2HPO_4$, pH 7,5 / 45 % Methanol in 10 mM $K_2HPO_4$, pH 7,5.

Den Säulenausfluß kontrolliert man mit dem analytischen HPLC-System - wie unten beschrieben - faßt die Methyl-Balhimycin-beinhaltenden Fraktionen zusammen, konzentriert sie im Vakuum und entsalzt sie durch Adsorption an das ®MCI-Gel CHP 20 P entsprechend Beispiel 6. Die Gefriertrocknung der phosphatfreien, reinen Antibiotikalösungen ergibt 11 mg Methyl-Balhimycin-Acetat in 98 %iger Reinheit.

Hochdruckflüssigkeitschromatographie (HPLC) Daten:

| | |
|---|---|
| Träger: | ®Lichrospher RP18, 5 µm, 250 x 4 $mm^2$ |
| Elutionsmittel: | 14 % Acetonitril in 0,1 %iger wässriger Trifluoressigsäure |
| Detektion: | UV-Absorption bei 210 nm |
| Retentionszeit: | 15,8 min, im Vergleich zu Balhimycin: 10,0 min |
| $[\alpha]_D^{24}$ = | - 59 ° (c = 1 % in Wasser) |

Beispiel 9: Hydrolytischer Abbau des Des-Methyl-Balhimycins zu Des-Methyl-des Gluco-Balhimycin

100 mg Des-Methyl-Balhimycin, gewonnen entsprechend dem Beispiel 3, werden in 2 ml 90 %iger Trifluoressigsäure gelöst und 70 Stunden bei Raumtemperatur reagieren lassen. Dann wird entsprechend Beispiel 6 aufgearbeitet. Man erhält 72 mg Des-Methyl-des-Gluco-Balhimycin-Acetat in 98 %iger Reinheit.

Hochdruckflüssigkeitschromatographie (HPLC) Daten:

| | |
|---|---|
| Träger: | $^R$Lichtrospher RP 18, 5µ, 250 x 4 $mm^2$ |
| Elutionsmittel: | 19 % Acetonitril in 0,1 %iger wässriger Trifluoressigsäure, |
| Detektion: | UV-Absorption bei 210 nm |
| Retentionszeit: | 9,1 min |

Beispiel 10: Gewinnung des Balhimycin R

150 mg des nach Beispiel 7 gewonnenen entsalzten und gefriergetrockneten Eluats R werden auf der gleichen Fractogelsäule wie im Beispiel 7 beschrieben, rechromatographiert. Das nun in 79 %iger Reinheit gewonnene Balhimycin R reinigt und entsalzt man weiter auf reverser Phase $^R$Nucleosil 10 $RP_{18}$ AB - wie Beispiel 7 - im 0,1 % Trifluoressigsäure-System. Das gefriergetrocknete Antibiotikum (52 mg) löst man in 3 ml Wasser, stellt den pH-Wert langsam auf 6 und fügt nach einsetzender Kristallisation noch 0,6 ml Ethanol zur Lösung. Nach Vervollständigung der Kristallisation wird zentrifugiert, das Kristallisat mit Ethanol gewaschen und im Vakuum getrocknet. Es resultieren 22 mg Balhimycin R in 99 %iger Reinheit.

Hochdruckflüssigkeitschromatographie (HPLC) Daten:

| | |
|---|---|
| Träger: | $^R$Lichrospher RP18, 5 µm, 250 x 4 $mm^2$ |
| Elutionsmittel: | 14 % Acetonitril in 0,1 %iger wässriger Trifluoressigsäure |
| Detektion: | UV-Absorption bei 210 nm |
| Retentionszeit: | 7,9 min, im Vergleich zu Balhimycin: 10,0 min |

Beispiel 11: Gewinnung des Ureido-Balhimycins aus Balhimycin

1500 mg Balhimycin gewonnen entsprechend EP 0 468 504, löst man in 60 ml Wasser, fügt 162 mg Kaliumcyanat hinzu, stellt den pH-Wert auf 6 ein und läßt die Lösung 2 Stunden stehen. Nach dieser Zeit wird durch präparative HPLC im 0,1 % Trifluoressigsäuresystem auf einer 500 ml fassenden $^R$Nucleosil 1015 C18 P, 250-2" Säule getrennt. Die Ureido-Balhimycin-haltigen Fraktionen sammelt man getrennt vom Balhimycin, gefriergetrocknet sie und kristallisiert sie in Wasser/Ethanol bei pH 5. Zentrifugation und Trocknung ergeben 1,3 g Ureido-Balhimycin in über 98 %iger Reinheit.

Beispiel 12: Gewinnung des Balhimycin V

Eine 100 ml Chromatographie-Säule wird mit $^R$Fractogel EMD-$SO_3$-Kationenaustauscher gefüllt und mit 25 mM Ammonium-formiat-Puffer, pH 4,2 (Puffer A) äquilibriert. Dann werden 1 g Roh-Balhimycin (II), gewonnen nach Beispiel 2, in 100 ml Wasser gelöst, auf pH 4 eingestellt, auf die Säule aufgetragen und mit 200 ml Puffer A nachgewaschen. Anschließend legt man einen 0,5 M Natriumchloridgradienten in Puffer A, pH 4 an. Zunächst werden die weniger basischen Antibiotika der Balhimycin-Reihe von der Säule eluiert, mit 0,34 - 0,36 M NaCl-Lösung des Balhimycin V. Die entsprechenden Balhimycin V-haltigen Fraktionen werden zusammengefaßt und wie im Beispiel 6 beschrieben auf 100 ml $^R$MCI-Gel CHP 20 P entsalzt und gefriergetrocknet. Sie ergeben 80 mg Balhimycin V-Acetat.

Hochdruckflüssigkeitschromatographie (HPLC) Daten:

| | |
|---|---|
| Träger: | $^R$Lichrospher RP, 18,5 µm, 250 x 4 $mm^2$ |
| Elutionsmittel: | 14 % Acetonitril in 0,1 %iger wässriger Trifluoressigsäure |
| Detektion: | UV-Absorption bei 210 nm |
| Retentionszeit: | 10,3 bis 10,4 min, als breiter Peak, Vergleich Balhimycin: 10 min |

Retentionszeit des Umsetzungsproduktes des Balhimycins V mit Kaliumcyanat nach Beispiel 10: 12,4 min
Vergleich Balhimycin: 10 min.

FAB-Massenspektrum:
Aus allen Molekül-Ionen berechnete Masse 1588 Da.

ESI Massenspektrum:
Aus allen Molekül-Ionen berechnete Massen.

MG 1588 Da(Diketonform), MG 1606 Da(Monohydrat), MG 1624 Da(Dihydrat)

## Patentansprüche

**Patentansprüche für folgende Vertragsstaaten : AT, BE, CH, DE, DK, FR, GB, IT, LI, LU, NL, PT, SE**

**1.** Des-Methyl-Balhimycin, eine Verbindung der Formel I,

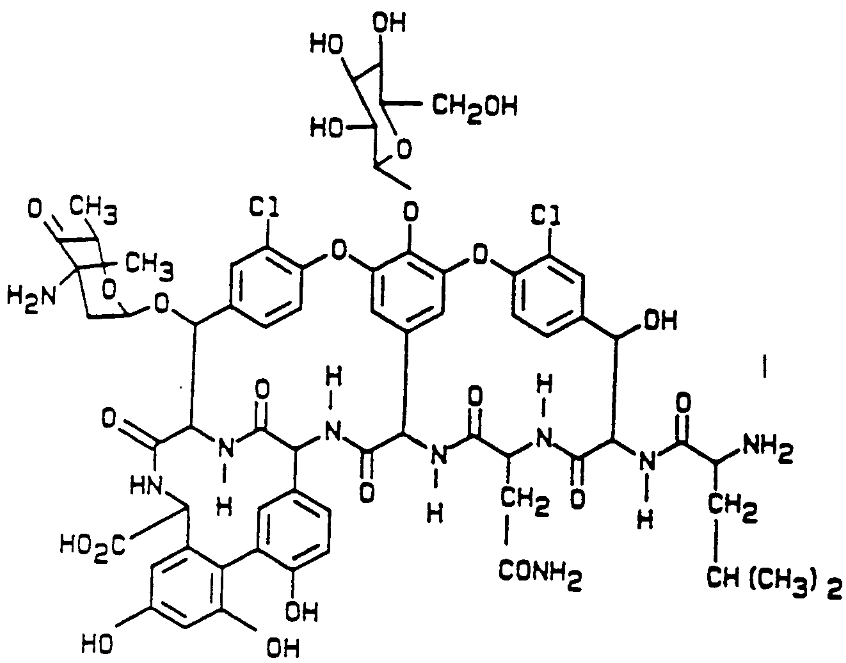

I

Des-Methylleucyl-Balhimycin, eine Verbindung der Formel II,

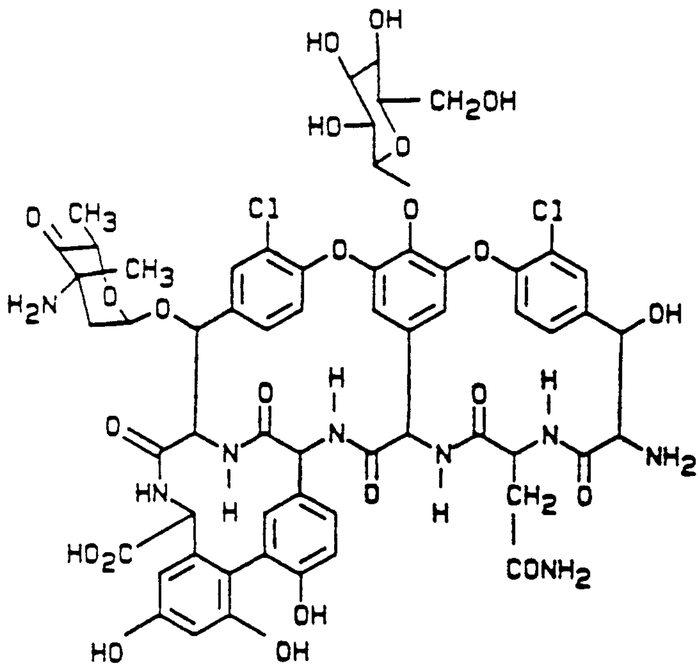

II

Des-Gluco-Balhimycin, eine Verbindung der Formel III,

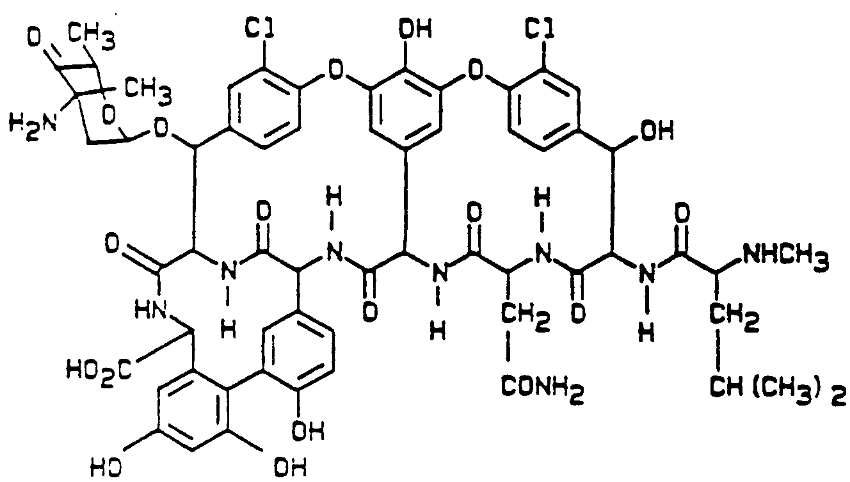

III

Ureido-Balhimycin, eine Verbindung der Formel IV,

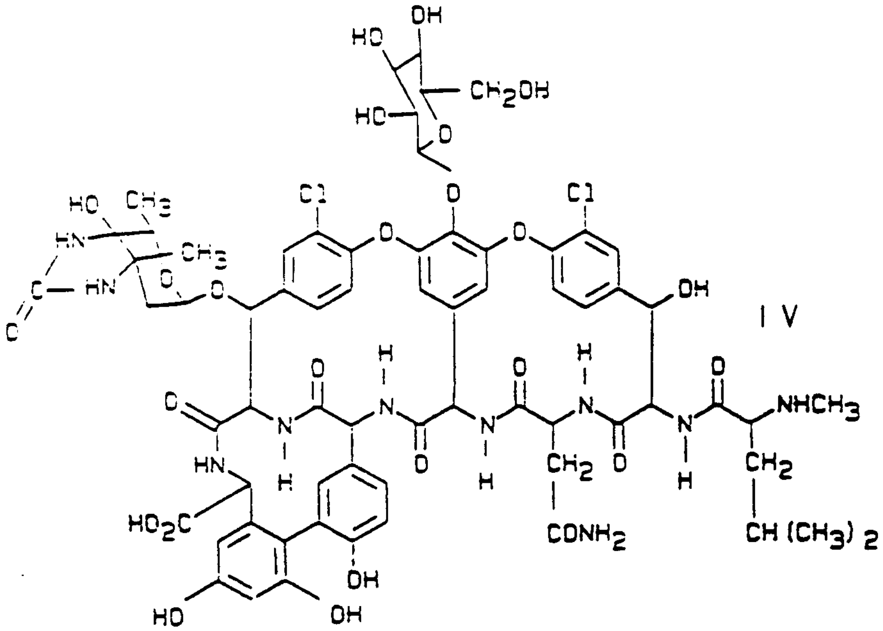

IV

Des-Methyl-des-Gluco-Balhimycin, eine Verbindung der Formel V,

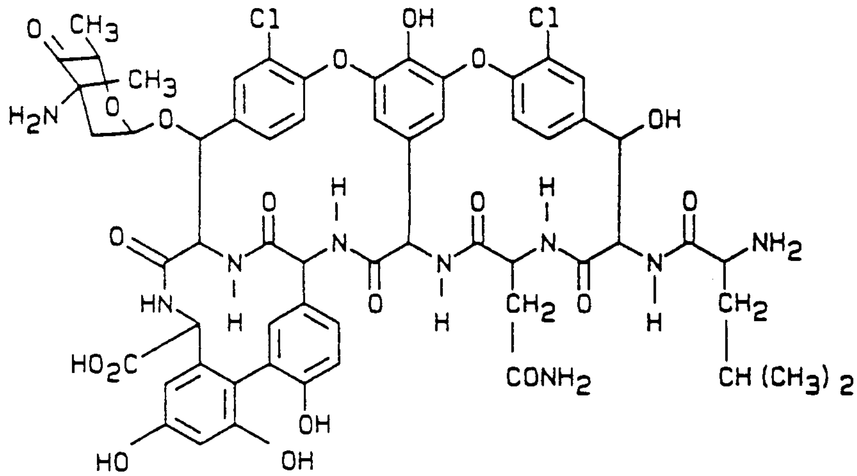

V

Methyl-Balhimycin, eine Verbindung der Formel $C_{67}H_{75}Cl_2N_9O_{24}$, Balhimycin R, eine Verbindung der Formel $C_{72}H_{83}Cl_2N_9O_{28}$ und Balhimycin V, eine Verbindung der Formel $C_{73}H_{84}Cl_2N_{10}O_{26}$ sowie deren Hydrate und physiologisch verträgliche Salze.

**2.** Verbindungen gemäß Anspruch 1 in kristalliner Form.

**3.** Verbindungen gemäß Anspruch 1, herstellbar durch Fermentation von Actinomyces species Y-86,21022 (DSM 5908) in einem wäßrigen Nährmedium und nachfolgende Isolierung.

**4.** Verfahren zur Herstellung von Verbindungen gemäß den Ansprüchen 1 bis 3, dadurch gekennzeichnet, daß der Mikroorganismus Actinomyces species Y-86,21022 (DSM 5908) in einem wäßrigen Nährmedium kultiviert wird und die Zielverbindungen anschließend isoliert und gereinigt werden.

**5.** Verfahren zur Herstellung von Des-Methylleucyl-Balhimycin durch Edman-Abbau von Balhimycin oder von Des-Methyl-Balhimycin.

6. Verfahren zur Herstellung von Des-Gluco-Balhimycins durch hydrolytische Spaltung von Balhimycin.

7. Verfahren zur Herstellung von Ureido-Balhimycin, dadurch gekennzeichnet, daß Balhimycin mit einem Isocyanat oder mit Harnstoff umgesetzt wird.

8. Verfahren zur Herstellung von Des-Methyl-des-Gluco-Balhimycin durch hydrolytische Spaltung von Des-Methyl-Balhimycin.

9. Arzneimittel, enthaltend eine wirksame Menge mindestens einer Verbindung gemaß den Ansprüchen 1 oder 2.

10. Verwendung einer Verbindung gemäß den Ansprüchen 1 oder 2 zur Herstellung von Arzneimitteln.

11. Verwendung einer Verbindung gemäß den Ansprüchen 1 oder 2 zur Herstellung von Arzneimitteln mit antibiotischer Wirkung.

12. Verfahren zur Herstellung von Arzneimitteln, dadurch gekennzeichnet, daß eine Verbindung gemäß den Ansprüchen 1 oder 2 ggf. mit pharmakologisch unbedenklichen Hilfs- und/oder Trägerstoffen in eine geeignete Darreichungsform gebracht wird.

13. Verwendung einer Verbindung gemäß den Ansprüchen 1 oder 2 als Wachstumsförderer in der Landwirtschaft.

**Patentansprüche für folgende Vertragsstaaten : ES, GR**

1. Verfahren zur Herstellung von Des-Methyl-Balhimycin, einer Verbindung der Formel I,

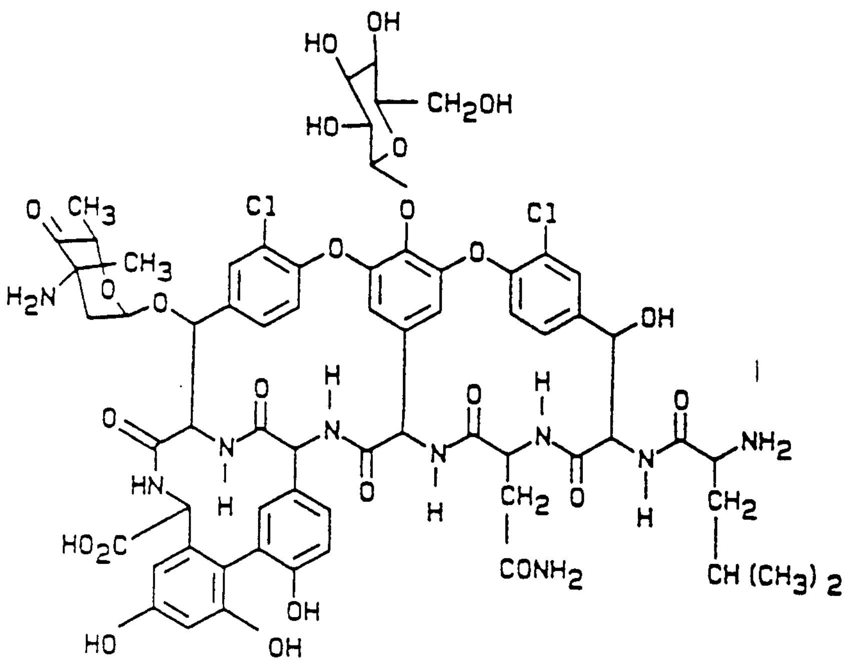

I

Des-Methylleucyl-Balhimycin, einer Verbindung der Formel II,

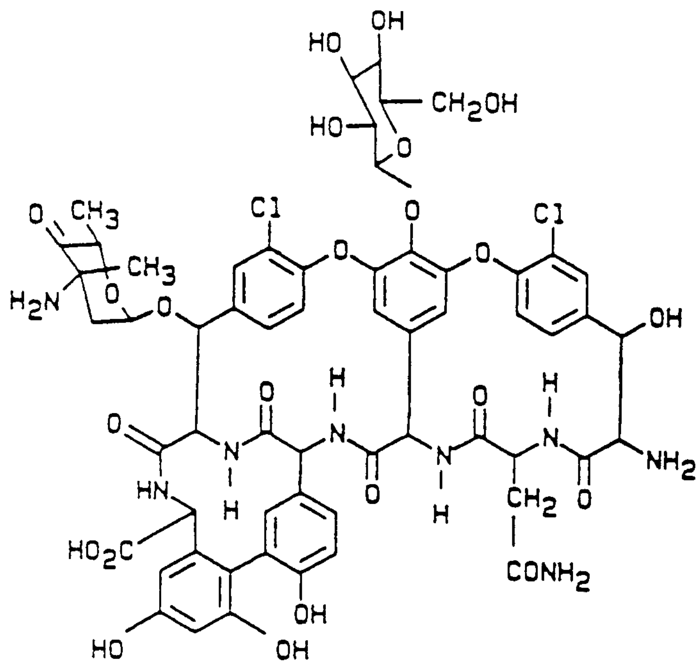

II

Des-Gluco-Balhimycin, einer Verbindung der Formel III,

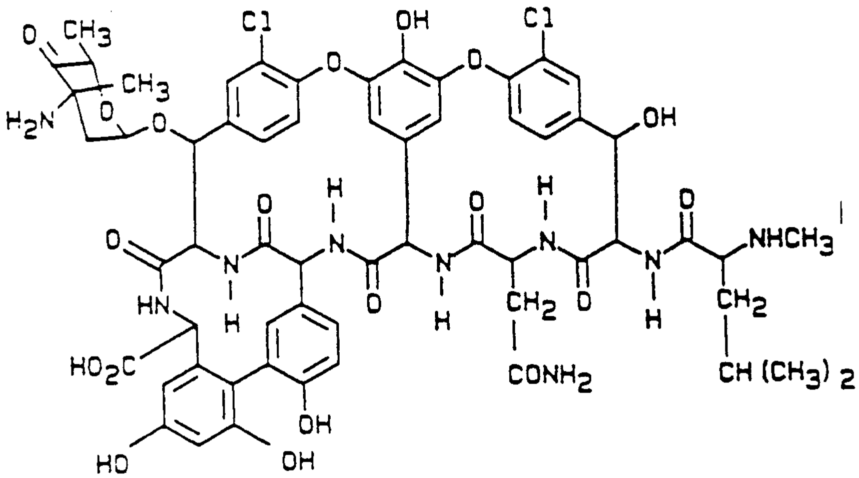

III

Ureido-Balhimycin, einer Verbindung der Formel IV,

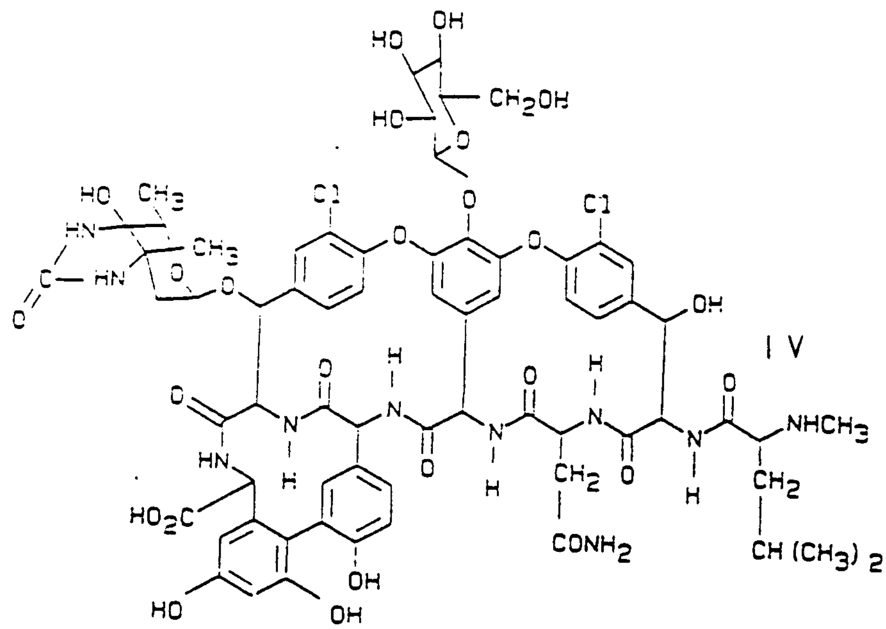

IV

Des-Methyl-des-Gluco-Balhimycin, einer Verbindung der Formel V,

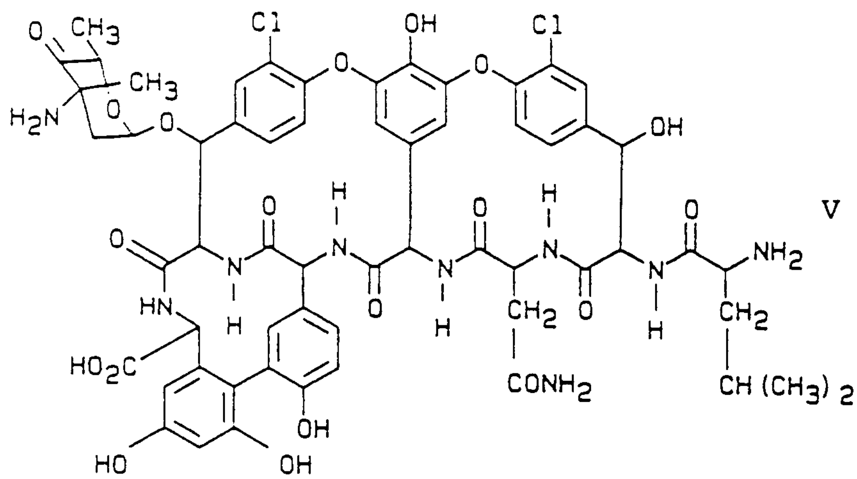

V

Methyl-Balhimycin, einer Verbindung der Formel $C_{67}H_{75}Cl_2N_9O_{24}$, Balhimycin R, einer Verbindung der Formel $C_{72}H_{83}Cl_2N_9O_{28}$ und Balhimycin V, einer Verbindung der Formel $C_{73}H_{84}Cl_2N_{10}O_{26}$ sowie deren Hydrate und physiologisch verträgliche Salze, dadurch gekennzeichnet, daß der Mikroorganismus Actinomyces species Y-86,21022 (DSM 5908) in einem wäßrigen Nährmedium kultiviert wird und die Zielverbindungen anschließend isoliert und gereinigt werden.

2. Verfahren zur Herstellung von Des-Methylleucyl-Balhimycin durch Edman-Abbau von Balhimycin oder von Des-Methyl-Balhimycin.

3. Verfahren zur Herstellung von Des-Gluco-Balhimycin durch hydrolytische Spaltung von Balhimycin.

4. Verfahren zur Herstellung von Ureido-Balhimycin, dadurch gekennzeichnet, daß Balhimycin mit einem Isocyanat oder mit Harnstoff umgesetzt wird.

5. Verfahren zur Herstellung von Des-Methyl-des-Gluco-Balhimycin durch hydrolytische Spaltung von Des-Methyl-Balhimycin.

6. Arzneimittel, enthaltend eine wirksame Menge mindestens einer Verbindung gemäß Anspruch 1.

7. Verwendung einer Verbindung gemäß Anspruch 1 zur Herstellung von Arzneimitteln.

8. Verwendung einer Verbindung gemäß Anspruch 1 zur Herstellung von Arzneimitteln mit antibiotischer Wirkung.

9. Verfahren zur Herstellung von Arzneimitteln, dadurch gekennzeichnet, daß eine Verbindung gemäß Anspruch 1 ggf. mit pharmakologisch unbedenklichen Hilfs- und/oder Trägerstoffen in eine geeignete Darreichungsform gebracht wird.

10. Verwendung einer Verbindung gemäß Anspruch 1 als Wachstumsförderer in der Landwirtschaft.

**Claims**

**Claims for the following Contracting States : AT, BE, CH, DE, DK, FR, GB, IT, LI, LU, NL, PT, SE**

1. Desmethylbalhimycin, a compound of the formula I,

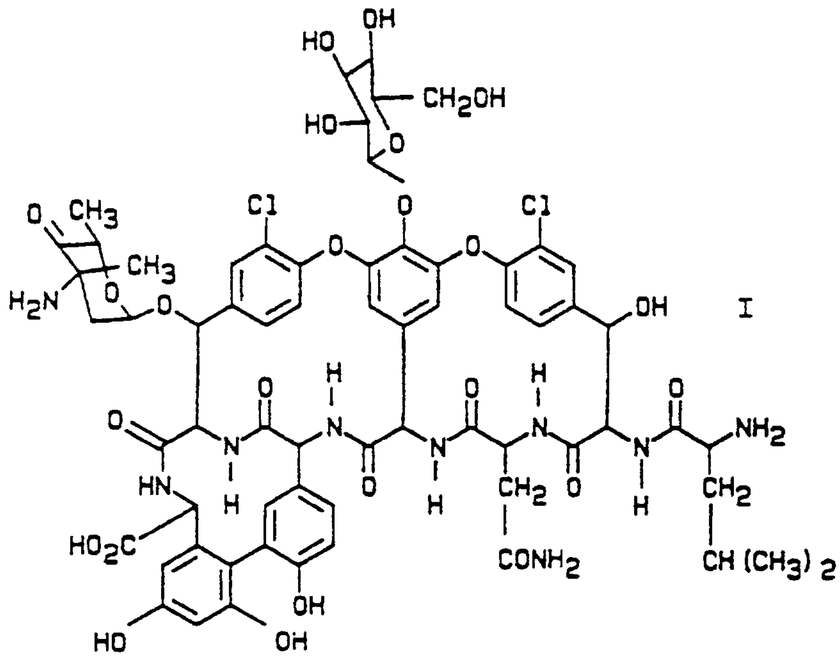

I

desmethylleucylbalhimycin, a compound of the formula II,

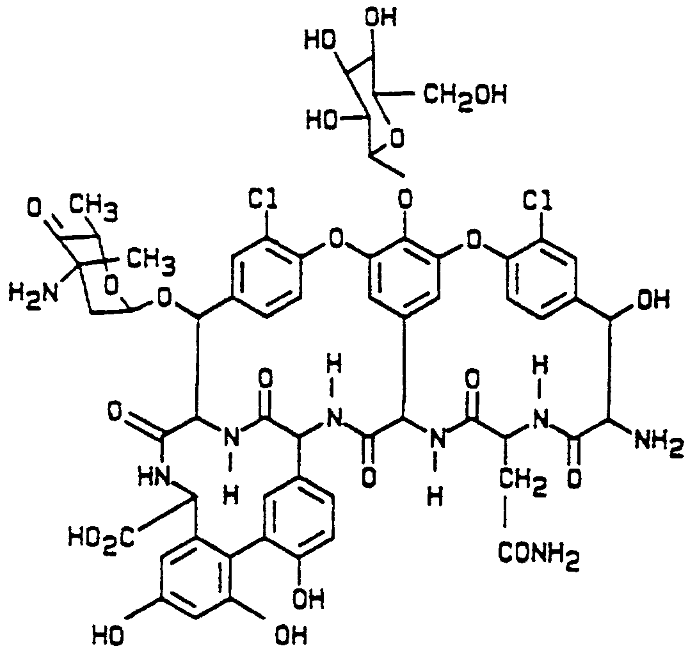

II

desglucobalhimycin, a compound of the formula III,

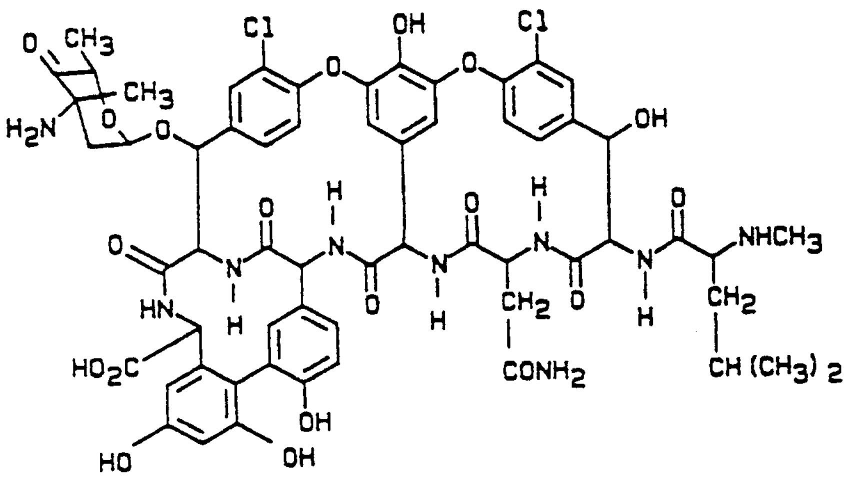

III

ureidobalhimycin, a compound of the formula IV,

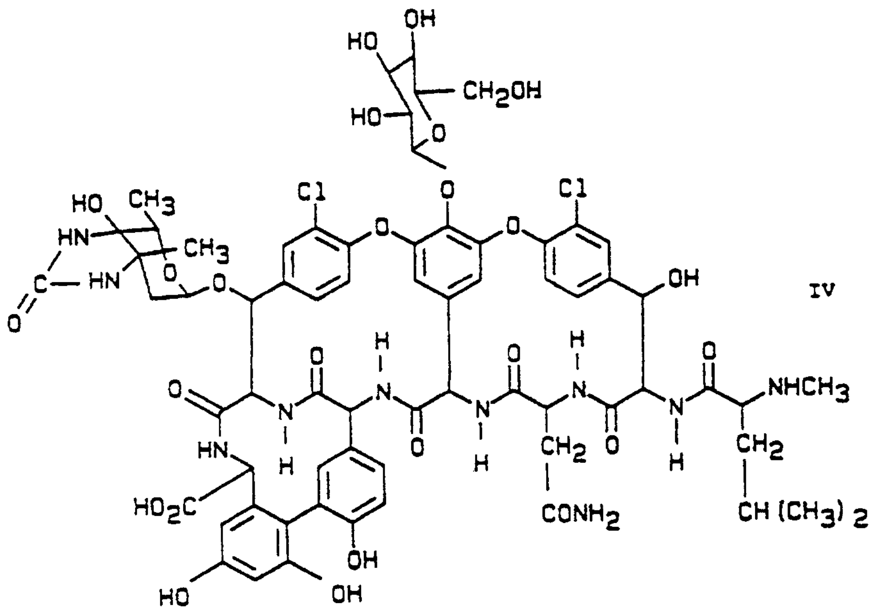

IV

desmethyldesglucobalhimycin, a compound of the formula V,

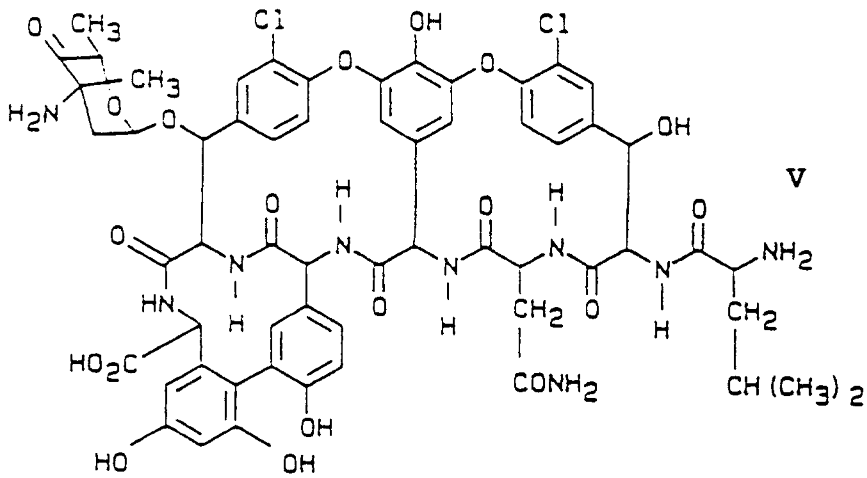

V

methylbalhimycin, a compound of the formula $C_{67}H_{75}Cl_2N_9O_{24}$, balhimycin R, a compound of the formula $C_{72}H_{83}Cl_2N_9O_{28}$ and balhimycin V, a compound of the formula $C_{73}H_{84}Cl_2N_{10}O_{26}$, and their hydrates and physiologically tolerable salts.

2. A compound as claimed in claim 1 in crystalline form.

3. A compound as claimed in claim 1, which can be prepared by fermentation of Actinomyces species Y-86,21022 (DSM 5908) in an aqueous nutrient medium and subsequent isolation.

4. A process for the preparation of compounds as claimed in claims 1 to 3, which comprises culturing the microorganism Actinomyces species Y-86,21022 (DSM 5908) in an aqueous nutrient medium and then isolating and purifying the target compounds.

5. A process for the preparation of desmethylleucylbalhimycin by Edman degradation of balhimycin or of desmethylbalhimycin.

6. A process for the preparation of desglucobalhimycin by hydrolytic cleavage of balhimycin.

7. A process for the preparation of ureidobalhimycin which comprises reacting balhimycin with an isocyanate or with urea.

8. A process for the preparation of desmethyldesglucobalhimycin by hydrolytic cleavage of desmethylbalhimycin.

9. A pharmaceutical containing an effective amount of at least one compound as claimed in claim 1 or 2.

10. The use of a compound as claimed in claim 1 or 2 for the production of pharmaceuticals.

11. The use of a compound as claimed in claim 1 or 2 for the production of pharmaceuticals having antibiotic action.

12. A process for the production of pharmaceuticals which comprises bringing a compound as claimed in claim 1 or 2 into a suitable administration form, if appropriate using pharmacologically acceptable auxiliaries and/or excipients.

13. The use of a compound as claimed in claim 1 or 2 as a growth promoter in agriculture.

**Claims for the following Contracting States : ES, GR**

1. A process for the preparation of desmethylbalhimycin, a compound of the formula I,

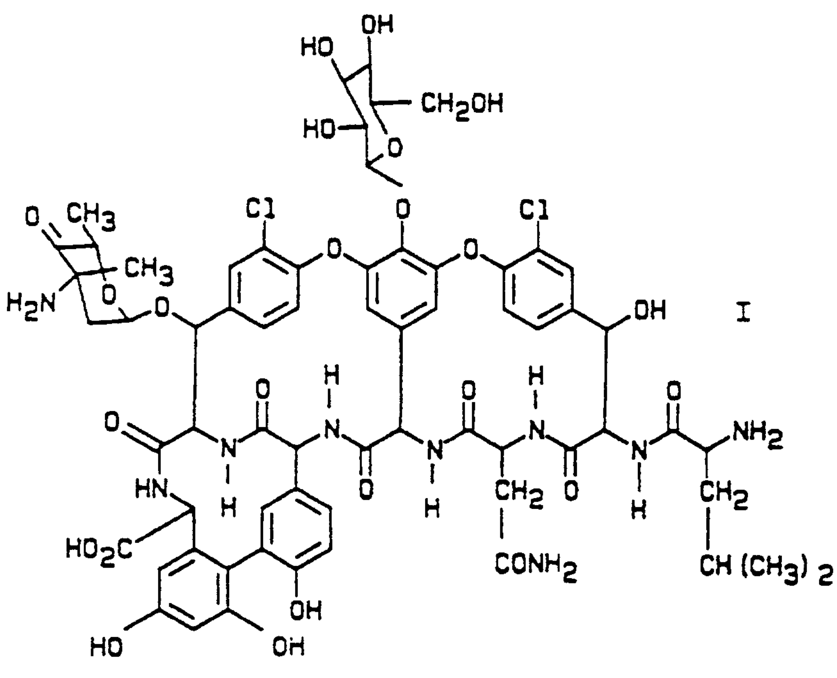

I

desmethylleucylbalhimycin, a compound of the formula II,

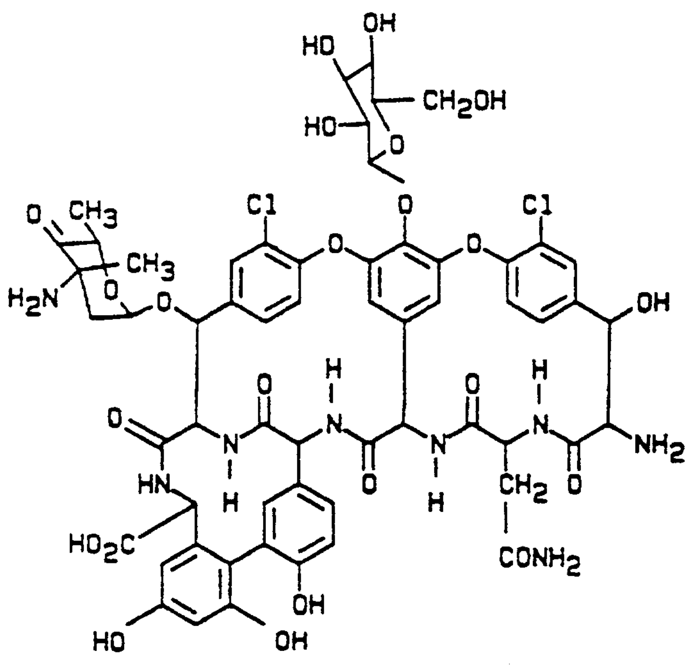

II

desglucobalhimycin, a compound of the formula III,

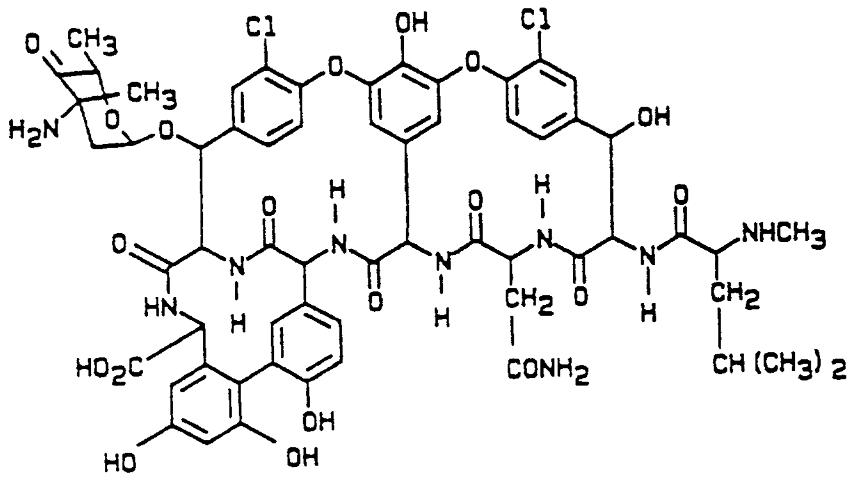

III

ureidobalhimycin, a compound of the formula IV,

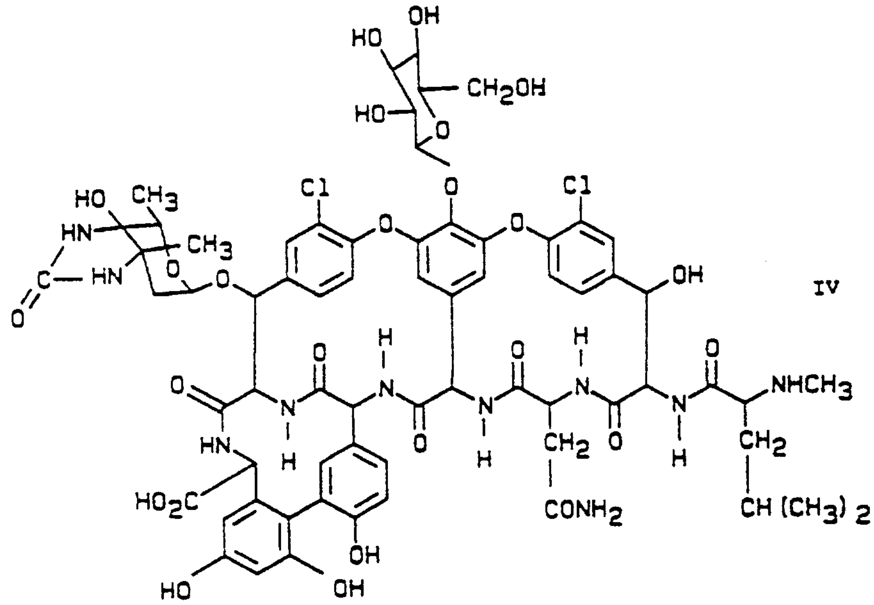

IV

desmethyldesglucobalhimycin, a compound of the formula V,

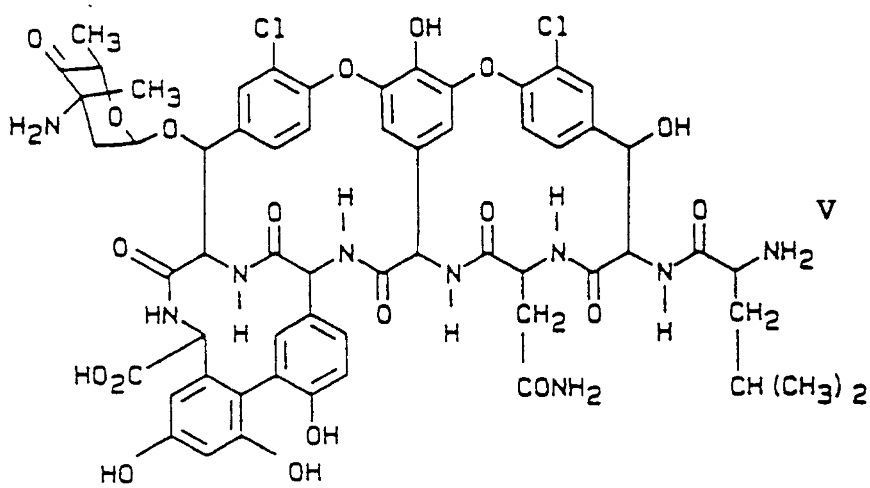

V

methylbalhimycin, a compound of the formula $C_{67}H_{75}Cl_2N_9O_{24}$, balhimycin R, a compound of the formula $C_{72}H_{83}Cl_2N_9O_{28}$ and balhimycin V, a compound of the formula $C_{73}H_{84}Cl_2N_{10}O_{26}$, and their hydrates and physiologically tolerable salts, which comprises culturing the microorganism Actinomyces species Y-86,21022 (DSM 5908) in an aqueous nutrient medium and then isolating and purifying the target compounds.

2. A process for the preparation of desmethylleucylbalhimycin by Edman degradation of balhimycin or of desmethylbalhimycin.

3. A process for the preparation of desglucobalhimycin by hydrolytic cleavage of balhimycin.

4. A process for the preparation of ureidobalhimycin which comprises reacting balhimycin with an isocyanate or with urea.

5. A process for the preparation of desmethyldesglucobalhimycin by hydrolytic cleavage of desmethylbalhimycin.

6. A pharmaceutical containing an effective amount of at least one compound as claimed in claim 1.

7. The use of a compound as claimed in claim 1 for the production of pharmaceuticals.

8. The use of a compound as claimed in claim 1 for the production of pharmaceuticals having antibiotic action.

9. A process for the production of pharmaceuticals which comprises bringing a compound as claimed in claim 1 into a suitable administration form, if appropriate using pharmacologically acceptable auxiliaries and/or excipients.

10. The use of a compound as claimed in claim 1 as a growth promoter in agriculture.

**Revendications**

**Revendications pour les Etats contractants suivants : AT, BE, CH, DE, DK, FR, GB, IT, LI, LU, NL, PT, SE**

1. Dé-méthyl-balhimycine, composé de formule I,

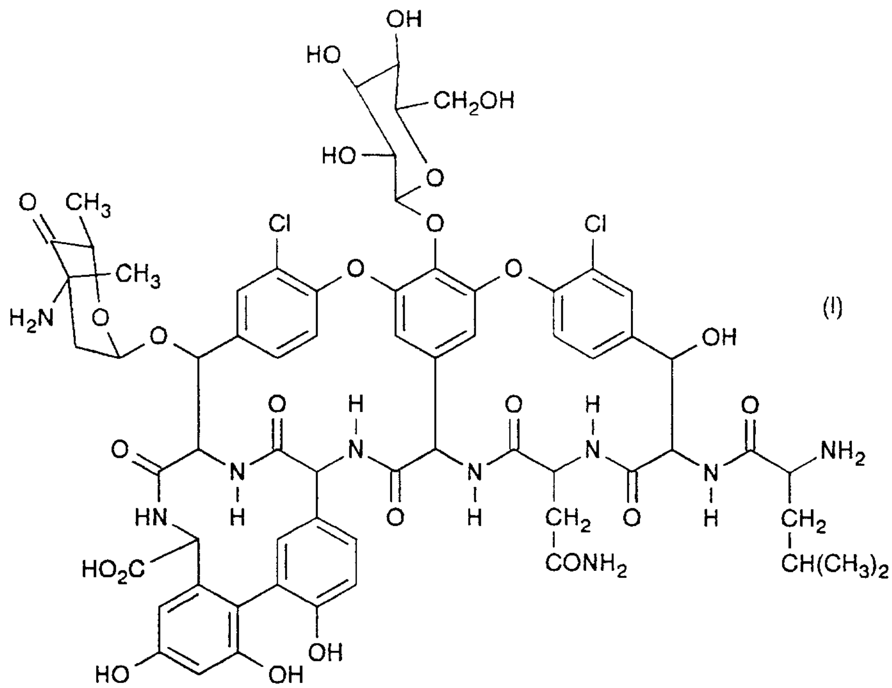

(I)

dé-méthylleucyl-balhimycine, composé de formule II,

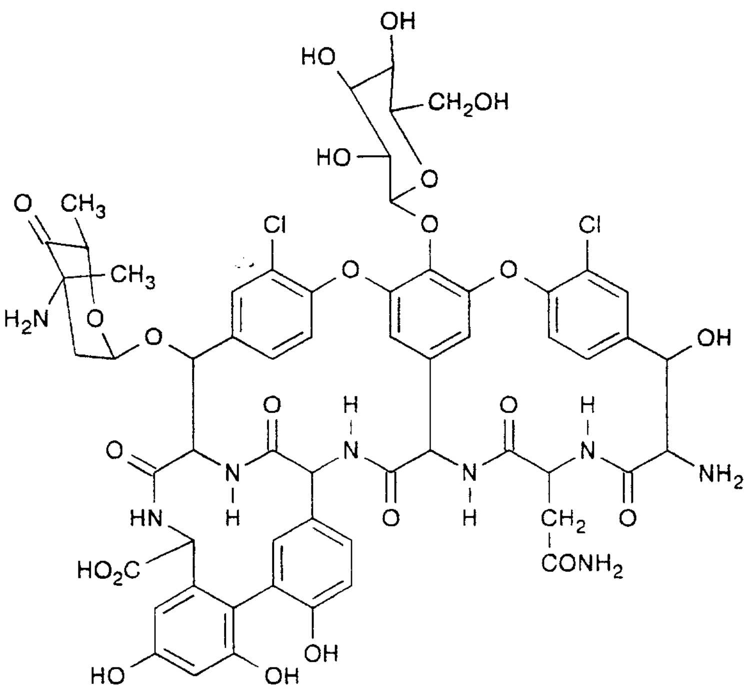

(II)

dé-gluco-balhimycine, composé de formule III,

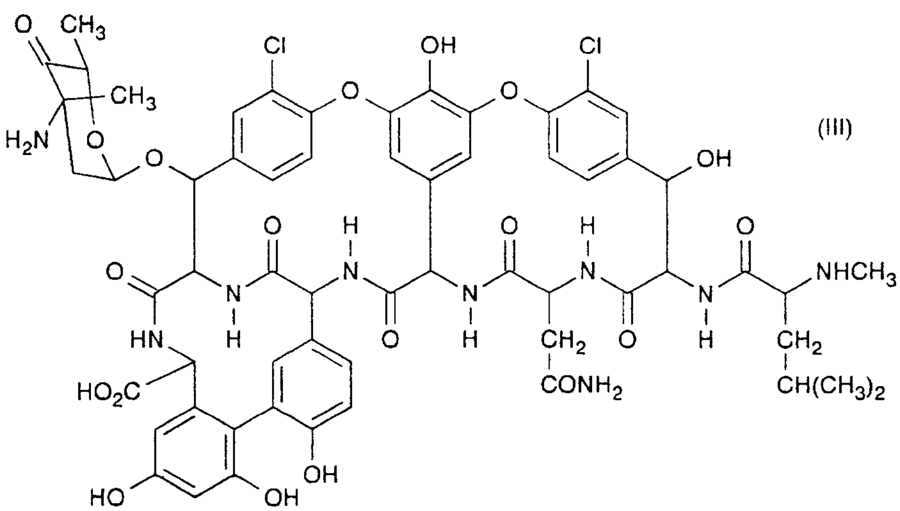

(III)

uréido-balhimycine, composé de formule IV,

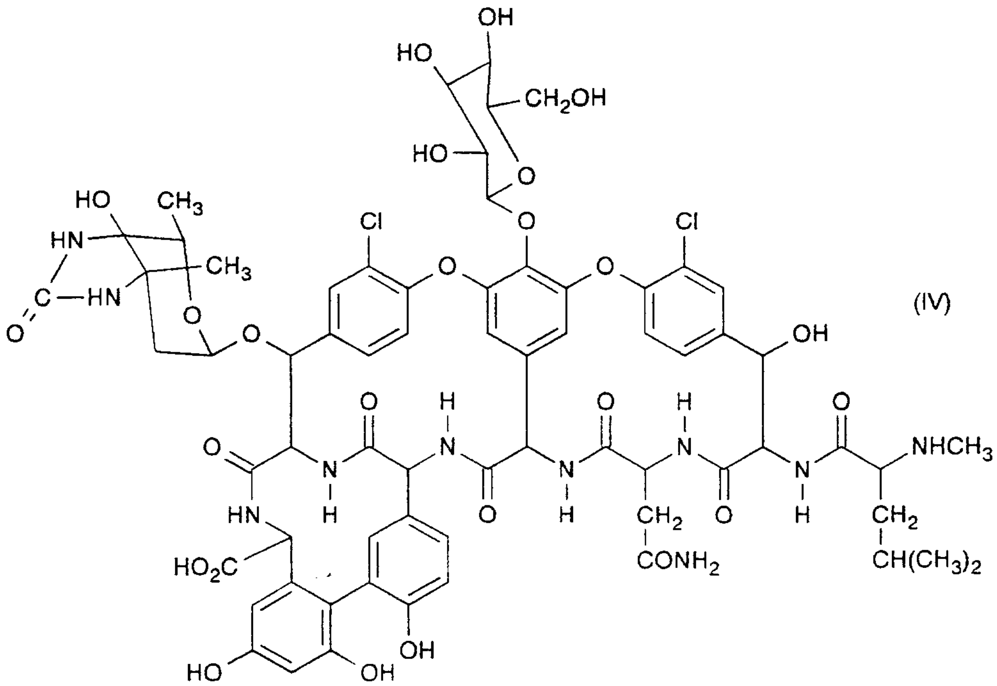

(IV)

dé-méthyl-dé-gluco-balhimycine, composé de formule V,

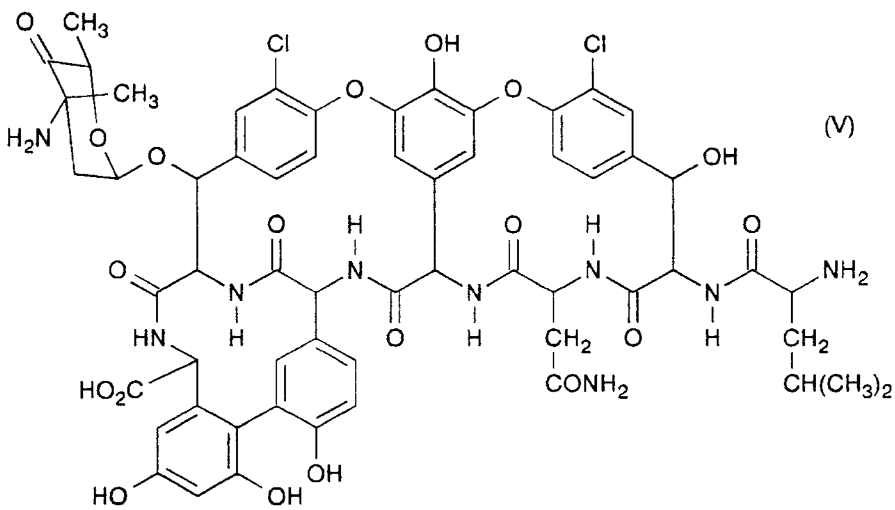

(V)

méthyl-balhimycine, composé de formule $C_{67}H_{75}Cl_2N_9O_{24}$, balhimycine R, composé de formule $C_{72}H_{83}Cl_2N_9O_{28}$ et balhimycine V, composé de formule $C_{73}H_{84}Cl_2N_{10}O_{26}$ ainsi que leurs hydrates et leurs sels physiologiquement acceptables.

**2.** Composés selon la revendication 1 sous forme cristalline.

**3.** Composés selon la revendication 1 que l'on peut préparer par fermentation de *Actinomyces spec.* Y-86,21022 (DSM 5908) dans un milieu nutritif aqueux puis isolement ultérieur.

**4.** Procédé pour la préparation des composés selon les revendicatons 1 à 3, caractérisé en ce que le micro-organisme *Actinomyces spec.* Y-86,21022 (DSM 5908) est cultivé dans un milieu nutritif aqueux et les composés cibles sont ensuite isolés et purifiés.

5. Procédé pour la préparation de dé-méthylleucyl-balhimycine par dégradation de Edman à partir de balhimycine ou de dé-méthyl-balhimycine.

6. Procédé pour la préparation de dé-gluco-balhimycine par clivage hydrolytique de balhimycine.

7. Procédé pour la préparation d'uréido-balhimycine, caractérisé en ce que l'on fait réagir la balhimycine avec un isocyanate ou avec de l'urée.

8. Procédé pour la préparation de dé-méthyl-dé-gluco-balhimycine par clivage hydrolytique de dé-méthyl-balhimycine.

9. Médicament contenant une quantité efficace d'au moins un composé selon la revendication 1 ou 2.

10. Utilisation d'un composé selon la revendication 1 ou 2 pour la préparation de médicaments.

11. Utilisation d'un composé selon la revendication 1 ou 2 pour la préparation de médicaments à activité antibiotique.

12. Procédé pour la préparation de médicaments, caractérisé en ce que l'on met un composé selon la revendication 1 ou 2, éventuellement avec des adjuvants et/ou des véhicules pharmacologiquement acceptables, sous une forme d'administration appropriée.

13. Utilisation d'un composé selon la revendication 1 ou 2 en tant que promoteur de croissance dans l'agriculture.

**Revendications pour les Etats contractants suivants : ES, GR**

1. Procédé pour la préparation de dé-méthyl-balhimycine, composé de formule I,

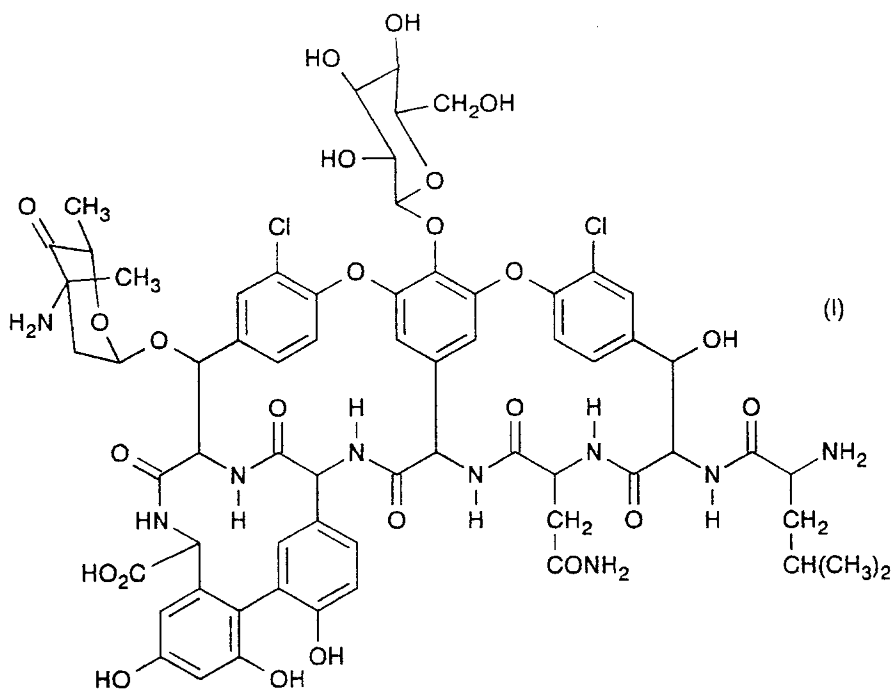

(I)

dé-méthylleucyl-balhimycine, composé de formule II,

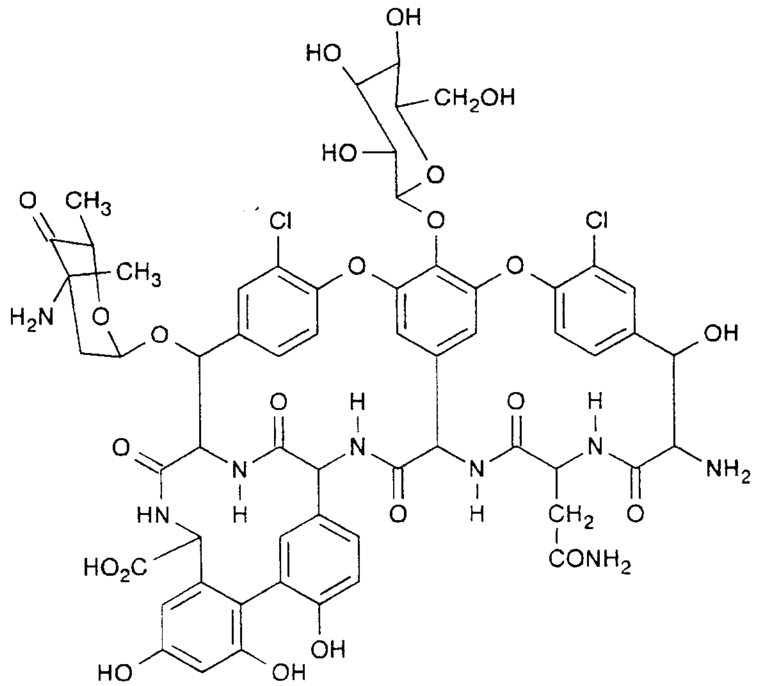

(II)

dé-gluco-balhimycine, composé de formule III,

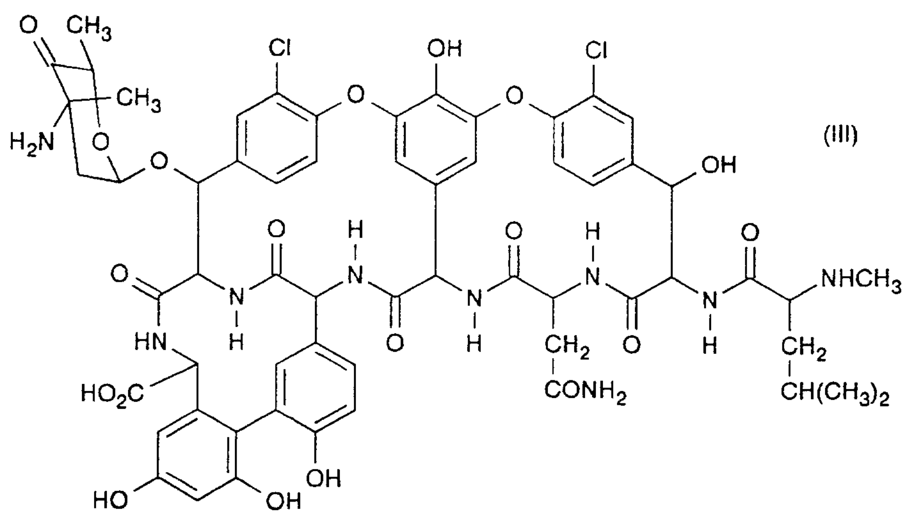

(III)

uréido-balhimycine, composé de formule IV,

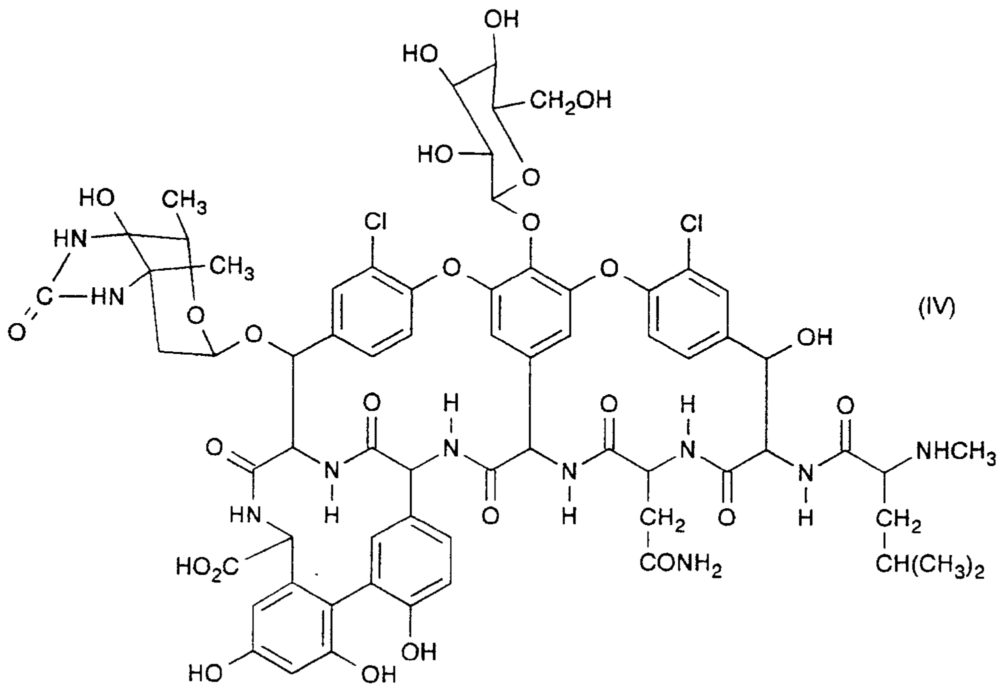

(IV)

dé-méthyl-dé-gluco-balhimycine, composé de formule V,

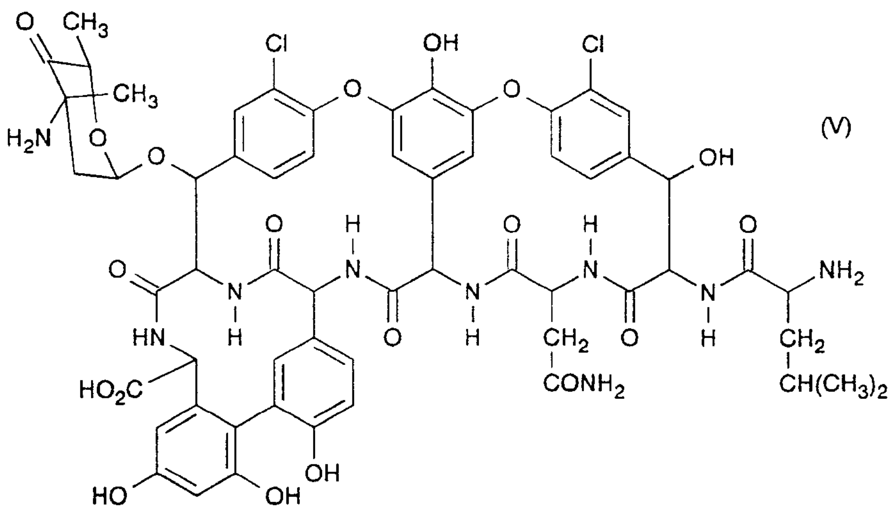

(V)

méthyl-balhimycine, composé de formule $C_{67}H_{75}Cl_2N_9O_{24}$, balhimycine R, composé de formule $C_{72}H_{83}Cl_2N_9O_{28}$ et balhimycine V, composé de formule $C_{73}H_{84}Cl_2N_{10}O_{26}$ ainsi que leurs hydrates et leurs sels physiologiquement acceptables, caractérisé en ce que le micro-organisme *Actinomyces spec.* Y-86,21022 (DSM 5908) est cultivé dans un milieu nutritif aqueux et les composés cibles sont ensuite isolés et purifiés.

**2.** Procédé pour la préparation de dé-méthylleucyl-balhimycine par dégradation de Edman à partir de balhimycine ou de dé-méthyl-balhimycine.

**3.** Procédé pour la préparation de dé-gluco-balhimycine par clivage hydrolytique de balhimycine.

**4.** Procédé pour la préparation d'uréido-balhimycine, caractérisé en ce que l'on fait réagir la balhimycine avec un isocyanate ou avec de l'urée.

5. Procédé pour la préparation de dé-méthyl-dé-gluco-balhimycine par clivage hydrolytique de dé-méthyl-balhimycine.

6. Médicament contenant une quantité efficace d'au moins un composé selon la revendication 1.

7. Utilisation d'un composé selon la revendication 1 pour la préparation de médicaments.

8. Utilisation d'un composé selon la revendication 1 pour la préparation de médicaments à activité antibiotique.

9. Procédé pour la préparation de médicaments, caractérisé en ce que l'on met un composé selon la revendication 1, éventuellement avec des adjuvants et/ou des véhicules pharmacologiquement acceptables, sous une forme d'administration appropriée.

10. Utilisation d'un composé selon la revendication 1 en tant que promoteur de croissance dans l'agriculture.